(19) Europäisches Patentamt
European Patent Office
Office européen des brevets

(11) Veröffentlichungsnummer: **0 425 925 B1**

(12) **EUROPÄISCHE PATENTSCHRIFT**

(45) Veröffentlichungstag der Patentschrift: **03.08.94**

(51) Int. Cl.5: **C07C 271/54**, C07C 271/22, A01N 47/22

(21) Anmeldenummer: **90120071.7**

(22) Anmeldetag: **19.10.90**

(54) **Substituierte Aminosäureamid-Derivate, deren Herstellung und Verwendung.**

(30) Priorität: **01.11.89 DE 3936298**

(43) Veröffentlichungstag der Anmeldung:
**08.05.91 Patentblatt 91/19**

(45) Bekanntmachung des Hinweises auf die Patenterteilung:
**03.08.94 Patentblatt 94/31**

(84) Benannte Vertragsstaaten:
**BE CH DE DK ES FR GB GR IT LI NL**

(56) Entgegenhaltungen:
**EP-A- 0 236 874**
**EP-A- 0 398 072**
**DE-A- 1 618 347**

**CHEMICAL ABSTRACTS, Band 109, Nr. 11, 12.September 1988, Seite 773, Zusammenfassung Nr. 93603f, Columbus, Ohio, US;& JP - A - 62252754**

(73) Patentinhaber: **BAYER AG**

**D-51368 Leverkusen(DE)**

(72) Erfinder: **Seitz, Thomas, Dr.**
**Mozartstrasse 32**
**W-4019 Monheim(DE)**
Erfinder: **Wollweber, Detlef, Dr.**
**Paul-Ehrlich-Strasse 17**
**W-5600 Wuppertal 1(DE)**
Erfinder: **Brandes, Wilhelm, Dr.**
**Eichendorffstrasse 3**
**W-5653 Leichlingen 1(DE)**

**EP 0 425 925 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte Aminosäureamid-Derivate und ein Verfahren zu deren Herstellung, sowie deren Verwendung in Schädlingsbekämpfungsmitteln.

Die erfindungsgemäßen Substanzen besitzen eine ausgezeichnete Wirkung bei der Bekämpfung von Schädlingen. Insbesondere können die erfindungsgemäßen Substanzen als Fungizide, vor allem im Pflanzenschutz, verwendet werden.

Bestimmte Aminosäureamide sind bereits bekannt, wie beispielsweise N-tert.-Butoxycarbonyl-L-leucylbenzylamid (EP-A-236 874).

Eine Verwendung dieser Verbindungen in Schädlingsbekämpfungsmitteln wird jedoch nicht beschrieben.

Gegenstand der vorliegenden Anmeldung sind somit neue Aminosäureamid-Derivate der allgemeinen Formel (I)

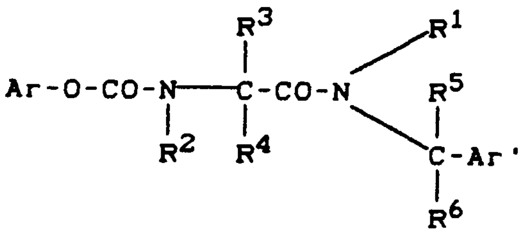

(I),

in welcher

| | |
|---|---|
| Ar und Ar′ | gleich oder verschieden sind und für unsubstituiertes oder substituiertes Aryl, unsubstituiertes oder substituiertes Aralkyl, unsubstituiertes oder substituiertes Heteroaryl und unsubstituiertes oder substituiertes Heteroarylalkyl stehen, |
| $R^1$, $R^2$, $R^3$, $R^4$, $R^5$ und $R^6$ | gleich oder verschieden sind und für Wasserstoff oder Alkyl stehen oder |
| $R^3$ und $R^4$ | zusammen mit dem Kohlenstoffatom, an das sie gebunden sind, einen Cycloalkylring bilden. |

Die Verbindungen der Formel (I) können außerdem ein oder mehrere Chiralitätszentren enthalten und können somit in verschiedene Enantiomeren-und Diastereomerengemischen vorliegen, die gegebenenfalls in üblicher Art und Weise getrennt werden können. Sowohl die reinen Enantiomeren und Diastereomeren, als auch die Gemische werden erfindungsgemäß beansprucht.

Im folgenden wird der Einfachheit halber stets von Verbindungen der Formel (I) gesprochen, obwohl sowohl die reinen Verbindungen, als auch die Gemische mit unterschiedlichen Anteilen an isomeren, enantiomeren und diastereomeren Verbindungen gemeint sind.

Die erfindungsgemäßen substituierten Aminosäureamid-Derivate sind durch die Formel (I) allgemein definiert.

Vorzugsweise bedeutet in den allgemeinen Formeln im folgenden, falls nicht anders definiert: Alkyl, einzeln oder in zusammengesetzten Resten

- geradkettiges oder verzweigtes Alkyl mit 1 bis 6, insbesondere 1 bis 4 Kohlenstoffatomen. Beispielhaft und vorzugsweise seien Methyl, Ethyl, n.- und i.-Propyl, n-, i-, s- und t-Butyl, genannt.

Aryl

- unsubstituiertes oder substituiertes Aryl mit 6 bis 10 Kohlenstoffatomen. Beispielhaft und vorzugsweise sei jeweils unsubstituiertes oder substituiertes Phenyl und Naphthyl, insbesondere unsubstituiertes oder substituiertes Phenyl genannt.

Aralkyl

- unsubstituiertes oder substituiertes Aralkyl mit 1 bis 4, insbesondere 1 oder 2 Kohlenstoffatomen im geradkettigen oder verzweigten Alkylteil und 6 bis 10 Kohlenstoffatomen, vorzugsweise Phenyl im Arylteil. Beispielhaft und vorzugsweise seien Benzyl, 1,1- und 1,2-Phenethyl und 1,1-, 1,2-, 1,3- und 2,2-Phenylpropyl genannt.

Heteroaryl

- unsubstituierter oder substituierter 5- bis 9-gliedriger Ring, insbesondere 5- bis 7-gliedriger Ring, der 1 bis 4, bevorzugt 1 bis 3, gleiche oder verschiedene Heteroatome enthält. Als Heteroatome seien vorzugsweise Sauerstoff, Schwefel und Stickstoff genannt. Beispielhaft und vorzugsweise seien

Pyrimidinyl, Pyrrolyl, Isothiazolyl, Oxazolyl, Thienyl, Furyl, Pyridazinyl, Pyrazinyl, Isooxazolyl, Thiazolyl und insbesondere Pyridyl genannt.

Heteroarylalkyl

- der Heteroarylteil entspricht den oben angegebenen Definitionen und Vorzugsbereichen. Der Alkylteil ist geradkettig oder verzweigt und enthält 1 bis 4 insbesondere 1 oder 2 Kohlenstoffatome. Beispielhaft und vorzugsweise seien Heteroarylmethyl, 1,1- und 1,2-Heteroarylethyl und 1,1-, 1,2-, 1,3- und 2,2-Heteroarylpropyl genannt.

Die gegebenenfalls substituierten Reste der allgemeinen Formeln können einen oder mehrere, vorzugsweise 1 bis 3, insbesondere 1 oder 2, gleiche oder verschiedene Substituenten tragen. Als Substituenten seien beispielhaft und vorzugsweise aufgeführt;

Alkyl mit vorzugsweise 1 bis 4, insbesondere 1 oder 2 Kohlenstoffatomen, wie Methyl, Ethyl, n.- und i.-Propyl und n.-, i.- und t.-Butyl; Alkoxy mit vorzugsweise 1 bis 4, insbesondere 1 oder 2 Kohlenstoffatomen, wie Methoxy, Ethoxy, n.- und i.-Propyloxy und n.-, i.-, sec.- und t.-Butyloxy; Alkylthio mit vorzugsweise 1 bis 4, insbesondere 1 oder 2 Kohlenstoffatomen, wie Methylthio, Ethylthio, n.- und i.-Propylthio und n.-, i.-, sec.- und t.-Butylthio; Halogenalkyl, Halogenalkoxy und Halogenalkylthio mit vorzugsweise 1 bis 4, insbesondere 1 oder 2 Kohlenstoffatomen und vorzugsweise 1 bis 9, insbesondere 1 bis 5 Halogenatomen, wobei die Halogenatome gleich oder verschieden sind und als Halogenatome, vorzugsweise Fluor, Chlor oder Brom, insbesondere Fluor stehen, wie Trifluormethyl, Difluormethyl, Pentafluorethyl, Tetrafluorethyl, Trifluorchlorethyl, Trifluorethyl, Trifluorethoxy, Difluormethoxy, Pentafluorethoxy, Tetrafluorethoxy, Trifluorchlorethoxy, Trifluormethoxy und Trifluormethylthio; Hydroxy; Halogen, vorzugsweise Fluor, Chlor, Brom und Jod, insbesondere Fluor, Chlor und Brom; Cyano; Nitro; Dialkylamino mit vorzugsweise 1 bis 4, insbesondere 1 oder 2 Kohlenstoffatomen je Alkylgruppe, wie Dimethylamino und Diethylamino; Carboxy; Alkylalkoxy mit 1 bis 4, insbesondere 1 oder 2 Kohlenstoffatomen in jedem Alkylteil; Carbonylalkoxy mit 1 bis 4, insbesondere 1 oder 2 Kohlenstoffatomen im Alkylteil, wie Carbonylmethoxy und Carbonylethoxy; Carbonylalkyl mit 1 bis 4, insbesondere 1 oder 2 Kohlenstoffatomen im Alkylteil, wie Acetyl und Propionyl; Formyl; Carbonylaryloxy mit 5 bis 10 Kohlenstoffatomen im Arylteil, wie Carbonylphenoxy; Carbonylaryl mit 6 bis 10 Kohlenstoffatomen im Arylteil, wie Benzoyl; Oxycarbonylalkyl mit 1 bis 4, insbesondere 1 oder 2 Kohlenstoffatomen im Alkylteil, wie Acetoxy; Oxycarbonylaryl mit 6 bis 10 Kohlenstoffatomen im Arylteil, wie Benzoyloxy; Carboxylamino, Carbonylaminoalkyl, Carbonylaminodialkyl, Aminocarbonyl, Alkylaminocarbonyl, Aminocarbonylalkyl und Alkylaminocarbonylalkyl mit jeweils 1 bis 4, insbesondere 1 oder 2 Kohlenstoffatomen im Alkylteil; Sulfonamido; Sulfonalkyl; Sulfonylalkyl und Sulfonylalkoxy mit jeweils 1 bis 4, insbesondere 1 oder 2 Kohlenstoffatomen; jeweils unsubstituiertes oder durch Halogen, insbesondere Fluor, Chlor und/oder Brom substituiertes Phenyl oder Phenoxy.

In den allgemeinen Formeln stehen $R^1$, $R^2$, $R^3$ und $R^5$ unabhängig voneinander bevorzugt für Methyl oder Ethyl und insbesondere für Wasserstoff.

Die hier aufgeführten Definitionen gelten in entsprechender Weise auch für die Definitionen in den folgenden bevorzugten Kombinationen von Resten.

Bevorzugt sind die Verbindungen der Formel (I), in welcher

| | |
|---|---|
| $R^1$, $R^2$, $R^3$, $R^4$, $R^5$ und $R^6$ | gleich oder verschieden sind und für Wasserstoff oder geradkettiges oder verzweigtes Alkyl mit 1 bis 6 Kohlenstoffatomen stehen oder |
| $R^3$ und $R^4$ | zusammen mit dem Kohlenstoffatom, an das sie gebunden sind, einen Cycloalkylring mit 3 bis 7 Kohlenstoffatomen bilden und |
| Ar und Ar′ | gleich oder verschieden sind und für jeweils unsubstituiertes oder substituiertes Phenyl, Furyl und Pyridyl oder unsubstituiertes oder im Phenylteil substituiertes Phenylalkyl mit 1 bis 4 Kohlenstoffatomen im geradkettigen oder verzweigten Alkylteil stehen, wobei als Substituenten im Phenylteil jeweils in Frage kommen: Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 9 Halogenatomen, wobei die Halogenatome gleich oder verschieden sind; Hydroxy; Halogen; Cyano; Nitro; Dialkylamino mit 1 bis 4 Kohlenstoffatomen je Alkylgruppe; Carboxy; Alkylalkoxy mit 1 bis 4 Kohlenstoffatomen in jedem Alkylteil; Carbonylalkoxy mit 1 bis 4 Kohlenstoffatomen im Alkylteil; Carbonylalkyl mit 1 bis 4 Kohlenstoffatomen im Alkylteil; Formyl; Carbonylaryloxy mit 5 bis 10 Kohlenstoffatomen im Arylteil; Carbonylaryl mit 6 bis 10 Kohlenstoffatomen im Arylteil; Oxycarbonylalkyl mit 1 bis 4 Kohlenstoffatomen im Alkylteil; Oxycarbonylaryl mit 6 bis 10 Kohlenstoffatomen im Arylteil; Carbonylamino, Carbonylaminoalkyl, Carbonylami- |

nodialkyl, Aminocarbonyl, Alkylaminocarbonyl, Aminocarbonylalkyl und Alkylaminocarbonylalkyl mit jeweils 1 bis 4 Kohlenstoffatomen im Alkylteil; Sulfonamido; Sulfonalkyl; Sulfonylalkyl und Sulfonylalkoxy mit jeweils 1 bis 4 Kohlenstoffatomen; jeweils unsubstituiertes oder durch Halogen substituiertes Phenyl oder Phenoxy.

Besonders bevorzugt sind diejenigen Verbindungen der Formel (I), in welcher

$R^1$ für Wasserstoff steht und

$R^2$, $R^3$ und $R^4$ gleich oder verschieden sind und für Wasserstoff oder geradkettiges oder verzweigtes Alkyl mit 1 bis 5 Kohlenstoffatomen stehen oder

$R^3$ und $R^4$ zusammen mit dem Kohlenstoffatom, an das sie gebunden sind, einen Cycloalkylring mit 3 bis 6 Kohlenstoffatomen bilden und

$R^5$ und $R^6$ gleich oder verschieden sind und für Wasserstoff, Methyl oder Ethyl stehen und

Ar für unsubstituiertes oder einfach bis dreifach, gleich oder verschieden substituiertes Phenyl steht, wobei die folgenden Substituenten in Frage kommen:
Alkyl, Alkoxy und Alkylthio mit jeweils 1 oder 2 Kohlenstoffatomen; Halogenalkyl, Halogenalkoxy und Halogenalkylthio mit 1 oder 2 Kohlenstoffatomen und 1 bis 5 Halogenatomen, wobei die Halogenatome gleich oder verschieden sind; Hydroxy; Fluor, Chlor, Brom und Jod; Cyano; Nitro; Dialkylamino mit 1 oder 2 Kohlenstoffatomen je Alkylgruppe; Carboxy; Alkylalkoxy mit 1 oder 2 Kohlenstoffatomen in jedem Alkylteil; Carbonylalkoxy mit 1 oder 2 Kohlenstoffatomen im Alkylteil; Carbonylalkyl mit 1 oder 2 Kohlenstoffatomen im Alkylteil; Formyl; Carbonylphenoxy; Benzoyl; Oxycarbonylalkyl mit 1 oder 2 Kohlenstoffatomen; Benzoyloxy; Carbonylamino, Carbonylaminoalkyl, Carbonylaminodialkyl, Aminocarbonyl, Alkylaminocarbonyl, Aminocarbonylalkyl und Alkylaminocarbonylalkyl mit jeweils 1 oder 2 Kohlenstoffatomen im Alkylteil; Sulfonamido; Sulfonalkyl, Sulfonylalkyl und Sulfonylalkoxy mit jeweils 1 oder 2 Kohlenstoffatomen; jeweils unsubstituiertes oder durch Fluor, Chlor oder Brom substituiertes Phenyl oder Phenoxy, und

Ar′ für jeweils unsubsituiertes oder einfach bis dreifach, gleich oder verschieden substituiertes Phenyl, Furyl oder Pyridyl oder für unsubstituiertes oder im Phenylteil einfach bis dreifach, gleich oder verschieden substituiertes Phenylalkyl mit 1 oder 2 Kohlenstoffatomen im Alkylteil steht, wobei als Phenylsubstituenten jeweils die oben genannten Phenylsubstituenten in Frage kommen.

Ganz besonders bevorzugt sind diejenigen Verbindungen der Formel (I), in welcher

$R^1$ für Wasserstoff steht und

$R^2$ für Wasserstoff oder Methyl steht und

$R^3$ und $R^4$ gleich oder verschieden sind und für Wasserstoff, Methyl, Ethyl, n- oder i-Propyl, n-, i-, s- oder t-Butyl oder 3-Pentyl stehen oder

$R^3$ und $R^4$ zusammen mit dem Kohlenstoffatom, an das sie gebunden sind, einen Cyclopropyl-, Cyclopentyl-oder Cyclohexylring bilden und

$R^5$ für Wasserstoff oder Methyl steht und

$R^6$ für Wasserstoff, Methyl oder Ethyl steht und

Ar für unsubstituiertes oder einfach bis zweifach, gleich oder verschieden substituiertes Phenyl steht, wobei die folgenden Substituenten in Frage kommen: Methyl, Ethyl, n- und i-Propyl, n-, i-, s-und t-Butyl, Methoxy, Ethoxy, i- und n-Propoxy, n-, i-, s- und t-Butoxy, Trifluormethyl, Difluormethyl, Pentafluorethyl, Tetrafluorethyl, Trifluorchlorethyl, Trifluorethyl, Trifluorethoxy, Difluormethoxy, Pentafluorethoxy, Tetrafluorethoxy, Trifluorchlorethoxy, Trifluormethoxy und Trifluormethylthio; Chlor, Brom, Fluor, Nitro und Cyano.

Ar′ für unsubstituiertes oder im Phenylteil einfach bis zweifach, gleich oder verschieden substituiertes Benzyl oder 1,2-Phenethyl steht, insbesondere jedoch für unsubstituiertes oder einfach bis zweifach, gleich oder verschieden substituiertes Phenyl steht, wobei als Phenylsubstituenten jeweils die oben genannten Phenylsubstituenten in Frage kommen.

Vor allem bevorzugt sind die substituierten Aminosäureamid-Derivate der allgemeinen Formel (I), in welcher

$R^1$, $R^2$, $R^5$, $R^6$, Ar und Ar′ die oben, insbesondere die in den Vorzugsbereichen, angegebenen Bedeutungen haben und

$R^3$ für Wasserstoff steht und

$R^4$ für Methyl, Ethyl, n-Propyl, t-Butyl, i-Butyl oder 3-Pentyl, insbesondere für i-Propyl oder s-Butyl steht oder

$R^3$ und $R^4$ zusammen mit dem Kohlenstoffatom, an das sie gebunden sind, einen Cycloalkylring mit 3 bis 6 Kohlenstoffatomen, insbesondere einen Cyclopropyl-, Cyclopentyl- oder Cyclohexylring, bilden.

Man erhält die substituierten Aminosäureamid-Derivate der allgemeinen Formel (I)

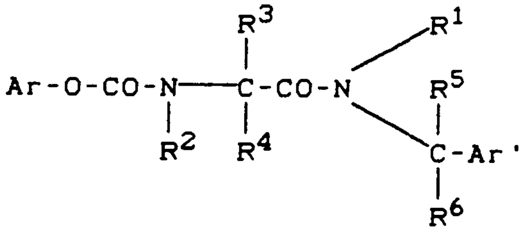

(I),

in welcher

$R^1$, $R^2$, $R^3$, $R^4$, $R^5$, $R^6$, Ar und Ar′ die oben angegebene Bedeutung haben,

wenn man

eine substituierte Aminosaure der Formel (11)

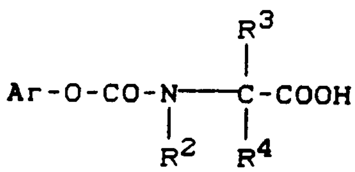

(II),

in welcher

Ar, $R^2$, $R^3$ und $R^4$ die oben angegebene Bedeutung haben, bzw. deren carboxy-aktivierte Derivate, gegebenenfalls in Gegenwart eines Katalysators, gegebenenfalls in Gegenwart eines Saurebindemittels und gegebenenfalls in Gegenwart eines Verdünnungsmittels

mit einem Amin der Formel (111)

$HNR^1\text{-}CR^5R^6Ar'$ (III),

in welcher

Ar′ , $R^1$, $R^5$ und $R^6$ die oben angegebene Bedeutung haben,

umsetzt.

Verwendet man beispielsweise Phenoxycarbonyl-L-Isoleucin und 4-Chlorphenethylamin als Ausgangsprodukte, so kann der Ablauf des erfindungsgemäßen Verfahrens durch das folgende Formelschema wiedergegeben werden:

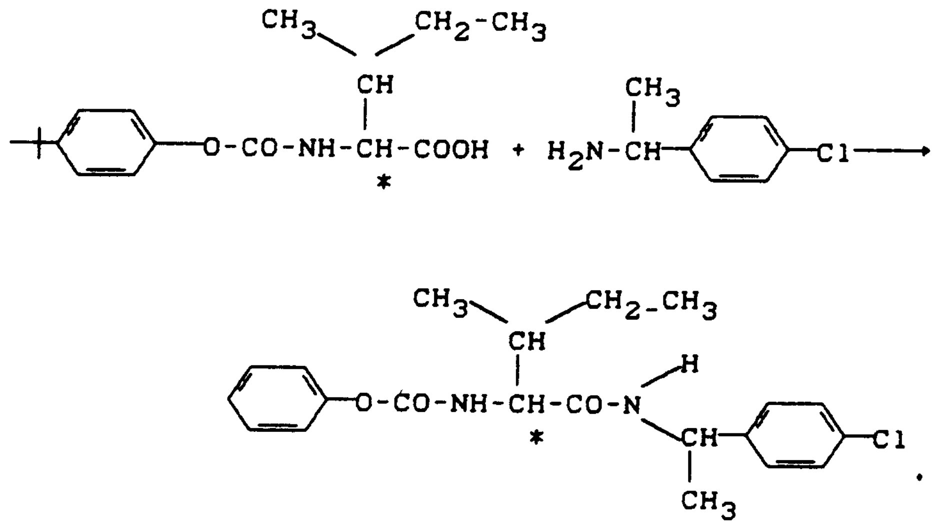

Die für die Durchführung des erfindungsgemäßen Verfahrens als Ausgangsstoffe zu verwendenden Aminosäure-Derivate sind durch die Formel (II) allgemein definiert. In dieser Formel haben Ar, $R^2$, $R^3$ und $R^4$ vorzugsweise die Bedeutungen, die bereits im Zusammenhang mit der Beschreibung der erfindungsmäßen Stoffe der Formel (I) für diese Substituenten bevorzugt genannt wurden.

Die Aminosäure-Derivate der Formel (II) sind allgemein bekannt (vgl. z.B. Houben-Weyl, Methoden der organischen Chemie, Band XV, Teil 1 und 2, Seiten 46 ff und 112 ff, Georg Thieme Verlag, Stuttgart 1974; D. Keller et.al., Org. Synth. 60, 2145 (1981); bzw. R.C. Sheppard, A Specialist Periodical Report, Aminoacids, Peptides and Proteins, The Royal Society of Chemistry, Burlington House, London 1978, bzw. I.P. Greenstein and M. Winitz, Chemistry of Amino Acids, I. Wiley Sons Inc., New York, London 1961; bzw. E. Schröder and K. Lübke, The Peptides Vol. I, Academic Press, New York, London 1965) oder können nach den dort angegebenen Verfahren erhalten werden.

Die außerdem für die Durchführung des erfindungsgemäßen Verfahrens als Ausgangsstoffe zu verwendenden carboxy-aktivierten Derivate der Aminosäure der Formel (II) sind allgemein bekannt.

Als carboxy-aktivierte Derivate der Aminosäuren der Formel (II) kommen alle Carboxy-aktivierten Derivate in Frage, wie Säurehalogenide, wie z.B. Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxysuccinimidester sowie mit Kondensationsmitteln, wie z.B. Dicyclohexylcarbodiimid oder Carbonyldiimidazol, in situ erzeugte aktivierte Formen der Aminosäuren.

Vorzugsweise werden die den Aminosäuren der Formel (II) entsprechenden Säurechloride und gemischten Anhydride eingesetzt. Sie können hergestellt werden, indem man die Aminosäuren der Formel (II) oder deren Salze mit einem Halogenierungsmittel oder einem der allgemein bekannten Mittel zur Herstellung von gemischten Anhydriden, wie beispielsweise Phosphorpentachlorid, Thionylchlorid, Oxalylchlorid oder Chlorameisensäureisobutylester, in allgemein bekannter Art und Weise umsetzt. Bevorzugt ist der Einsatz von Chlorameisensäureisobutylester.

Die Umsetzung kann in Gegenwart indifferenter Verdünnungsmittel wie z.B. aromatische, nichtaromatische oder halogenierte Kohlenwasserstoffe wie: Ketone, wie z.B. Aceton; Ester, wie z.B. Ethylacetat; Amide, wie z.B. Dimethylformamid, Nitrile, wie z.B. Acetonitril, Chlorkohlenwasserstoffe, wie z.B. Methylenchlorid, Kohlenwasserstoffe, wie z.B. Toluol; oder Ether, wie z.B. Tetrahydrofuran bzw. deren Mischungen und/oder in Gegenwart eines Säurebindemittels, wie vorzugsweise eines tertiären Amins, wie z.B. Triethylamin, Pyridin oder N-Methylpiperidin, bei Temperaturen von -78 °C bis 100 °C, vorzugsweise -60 °C bis 25 °C, durchgeführt werden.

Die außerdem für die Durchführung des erfindungsgemäßen Verfahrens als Ausgangsstoffe zu verwendenden Amine sind durch die Formel (III) allgemein definiert. In dieser Formel haben $R^1$, Ar′, $R^5$ und $R^6$ die oben genannten Bedeutungen.

Die Amine der Formel (III) sind allgemein bekannte Verbindungen der organischen Chemie.

Als Verdünnungsmittel kommen für das erfindungsgemäße Verfahren inerte, organische Lösungsmittel wie: Ketone, wie Aceton oder Ethylmethylketon; Ester wie Ethyl- oder Methylacetat; Amide wie Dimethylformamid; Nitrile, wie Acetonitril; Chlorkohlenwasserstoffe, wie Methylenchlorid oder Tetrachlorkohlenstoff; Kohlenwasserstoffe, wie Toluol oder Ether, wie Tetrahydrofuran sowie gegebenenfalls Wasser und deren

Mischungen in Frage.

Als Säurebindemittel kommen für das erfindungsgemäße Verfahren übliche anorganische und organische Säurebinder in Frage. Hierzu gehoren vorzugsweise tertiäre Amine, wie Triethylamin, Pyridin oder N-Methylpiperidin, sowie anorganische Basen, z.B. Metallhydroxide wie Natrium- und Kaliumhydroxid oder Metallcarbonate wie Natriumcarbonat oder Calciumcarbonat.

Das erfindungsgemäße Verfahren wird gegebenenfalls in Gegenwart eines Katalysators durchgeführt. Beispielsweise genannt seien 4-Dimethylaminopyridin, 1-Hydroxy-benzotriazol oder Dimethylformamid.

Die Temperaturen können bei der Durchführung des Verfahrens in einem größeren Bereich variiert werden. Im allgemeinen arbeitet man bei -78 bis +120 °C, vorzugsweise bei -60 bis +40 °C.

Bei der Durchführung des erfindungsgemäßen Verfahrens arbeitet man vorzugsweise in äquimolaren Mengen.

Dabei werden die Aminosäurederivate der Formel (II) als reine optische Isomere (D bzw. L-Form) oder als Racemate eingesetzt.

Die Erfindung umfaßt sowohl die reinen Isomeren als auch die Gemische. Diese Gemische können nach gebräuchlichen Methoden, z.B. selektive Kristallisation aus geeigneten Lösungsmitteln oder Chromatographie an Kieselgel oder Aluminiumoxid in die Komponenten aufgetrennt werden. Racemate können nach üblichen Methoden in die einzelnen Enantiomeren aufgetrennt werden, so z.B. durch Salzbildung mit optisch aktiven Säuren wie Camphersulfonsäure oder Dibenzoylweinsäure und selektive Kristallisation oder durch Derivatisierung mit geeigneten, optisch aktiven Reagenzien, Trennung der diastereomeren Derivate und Rückspaltung oder Trennung an optisch aktivem Säulenmaterial.

Die erfindungsgemäßen Wirkstoffe der Formel (I) weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum; Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg protektiv und systemisch zur Bekämpfung von Phytophthora-Arten an Tomaten oder Plasmopara-Arten an Reben sowie zur Bekämpfung von Reiskrankheiten, wie beispielsweise gegen den Erreger der Reisfleckenkrankheit (Pyricularia oryzae) oder gegen den Erreger der Reisstengelkrankheit (Pellicularia sasakii) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächen-aktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle; wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut von Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1

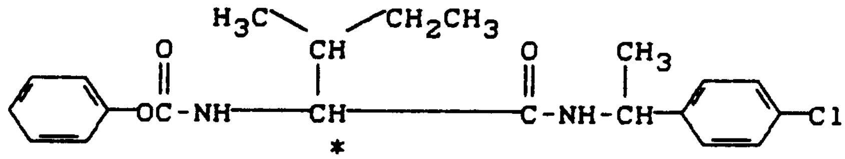

2,12 g (8,4 mmol) Phenoxycarbonyl-L-isoleucin werden in 50 ml THF/$CH_2Cl_2$ (1:9) gelöst und bei -20 °C mit 0,83 g (8,4 mmol) N-Methylpiperidin versetzt. Nach 5 Minuten Rühren bei -20 °C läßt man 1,15 g (8,4 mmol) Chlorameisensäureisobutylester zutropfen, rührt 10 Minuten bei -20 °C nach, kühlt auf -60 °C ab und gibt 1,30 g (8,4 mmol) 1-(4-Chlorphenyl)ethylamin gelöst in 5 ml THF/$CH_2Cl_2$ (1:9) zu. Es wird 2 Stunden bei -15 °C nachgerührt und danach weitere 15 Stunden bei Raumtemperatur. Zur Aufarbeitung wird abfiltriert, die Lösung im Vakuum eingeengt und der Rückstand in $CH_2Cl_2$ aufgenommen. Die organische Phase wird nacheinander mit Wasser, $NaHCO_3$-Lösung und Wasser gewaschen, über $Na_2SO_4$ getrocknet und im Vakuum vom Lösungsmittel befreit. Man erhält 2,30 g (71% d.Th.) farbloses $N^2$-Phenoxycarbonyl-$N^1$-[rac.-1-(4-chlorphenyl)ethyl]-L-isoleucinamid mit einem Schmelzpunkt von 150-156 °C.

Analog Beispiel 1 werden die folgenden Verbindungen der Formel (I) erhalten

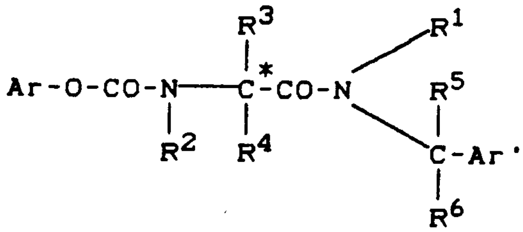

(I)

Tabelle 1

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 2 | 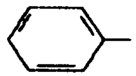 | H | H | $-CH(CH_3)_2$ | H | H | $CH_3$ | 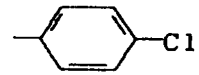 | 101-102° C | L |
| 3 | 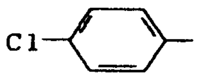 | H | H | $-CH(CH_3)_2$ | H | H | H | 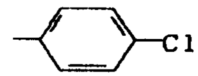 | 191-192° C | L |
| 4 | 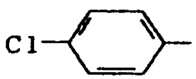 | H | H | $-CH(CH_3)_2$ | H | H | $CH_3$ | 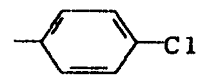 | 154-155° C | L |
| 5 | 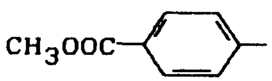 | H | H | $-CH(CH_3)_2$ | H | H | H | 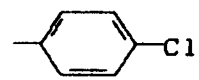 | 192-193° C | L |
| 6 | 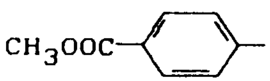 | H | H | $-CH(CH_3)_2$ | H | $CH_3$ | H | 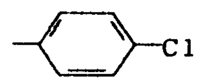 | 154-155° C | L |

<u>Tabelle 1</u> Fortsetzung

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 7 | 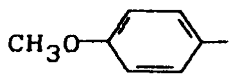 | H | H | $-CH(CH_3)_2$ | H | H | $CH_3$ | 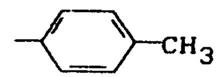 | 178-182° C | L |
| 8 | 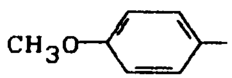 | H | H | $-CH(CH_3)_2$ | H | H | $CH_3$ | 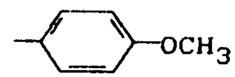 | 161-165° C | L |
| 9 | 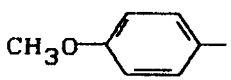 | H | H | $-CH(CH_3)_2$ | H | H | $CH_3$ | 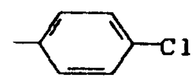 | 183-185° C | L |
| 10 | 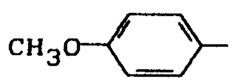 | H | H | $-CH(CH_3)-CH_2-CH_3$ | H | H | $CH_3$ | 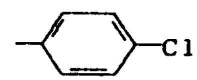 | 168-170° C | L |
| 11 | 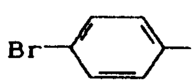 | H | H | $-CH(CH_3)_2$ | H | H | $CH_3$ | 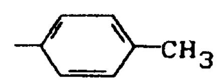 | 162-163° C | L |

<u>Tabelle 1</u> Fortsetzung

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 12 | 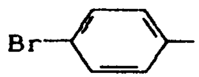 | H | H | $-CH(CH_3)_2$ | H | H | $CH_3$ | 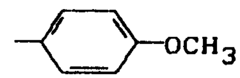 | 163-165° C | L |
| 13 | 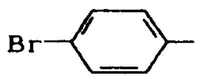 | H | H | $-CH(CH_3)_2$ | H | H | $CH_3$ | 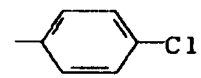 | 164-166° C | L |
| 14 | 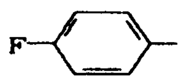 | H | H | $-CH(CH_3)_2$ | H | H | $CH_3$ | 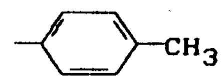 | 169-170° C | L |
| 15 | 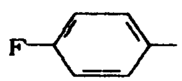 | H | H | $-CH(CH_3)_2$ | H | H | $CH_3$ | 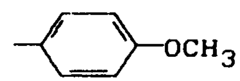 | 172-173° C | L |
| 16 | 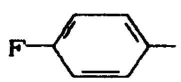 | H | H | $-CH(CH_3)_2$ | H | H | $CH_3$ | 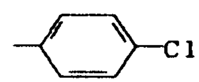 | 165-168° C | L |

Tabelle 1 Fortsetzung

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 17 | 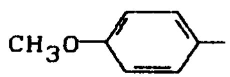 | H | H | $-CH(CH_3)_2$ | H | H | $CH_3$ | 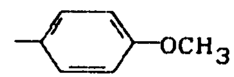 | 163-168° C | D/L |
| 18 | 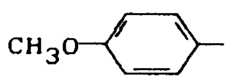 | H | H | $-CH(CH_3)_2$ | H | H | $CH_3$ | 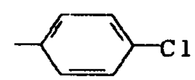 | 134-135° C | D/L |
| 19 | 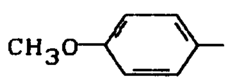 | H | H | $-CH(CH_3)-CH_2-CH_3$ | H | H | $CH_3$ | 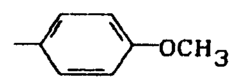 | 159-163° C | L |
| 20 | 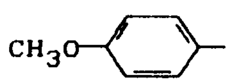 | H | H | $-CH(CH_3)_2$ | H | H | $-CH_2-CH_3$ | 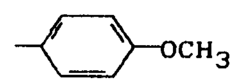 | 149-151° C | D/L |
| 21 | 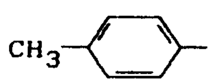 | H | H | $-CH(CH_3)_2$ | H | H | $CH_3$ | 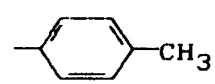 | 179-182° C | L |

Tabelle 1 Fortsetzung

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 22 | 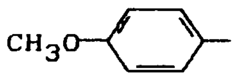 | H | H | $-CH-CH_2-CH_3$<br>\|<br>$CH_3$ | H | H | $CH_3$ | 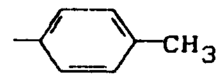 | 168-170° C | L |
| 23 | 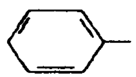 | H | H | $-CH(CH_3)_2$ | H | H | $CH_3$ | 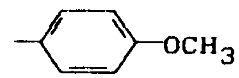 | 170-175° C | L |
| 24 | 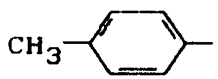 | H | H | $-CH(CH_3)_2$ | H | H | $-CH_2CH_3$ | 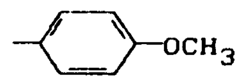 | 170-175° C | L |
| 25 | 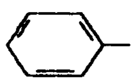 | H | H | $-CH(CH_3)_2$ | H | H | $-CH_2CH_3$ | 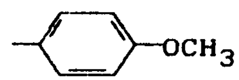 | 172-176° C | L |
| 26 | 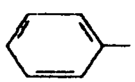 | H | H | $-CH(CH_3)_2$ | H | H | $CH_3$ | 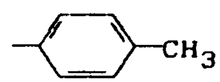 | 170-175° C | L |

**Tabelle 1** Fortsetzung

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 27 | 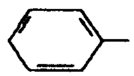 | H | H | $-CH(CH_3)_2$ | H | H | $CH_3$ | 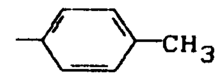 | 158-162° C | D/L |
| 28 | 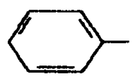 | H | H | $-CH(CH_3)_2$ | H | H | $CH_3$ | 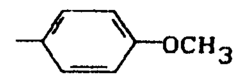 | 158-162° C | D/L |
| 29 | 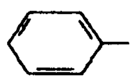 | H | H | $-CH(CH_3)_2$ | H | H | $CH_3$ | 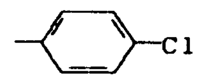 | 175-179° C | D/L |
| 30 | 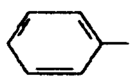 | H | H | $-CH(CH_3)_2$ | H | H | $-CH_2CH_3$ | 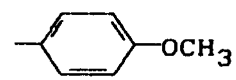 | 158-162° C | D/L |
| 31 | 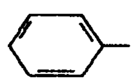 | H | H | $-CH-CH_2-CH_3$<br>$\vert$<br>$CH_3$ | H | H | $CH_3$ | 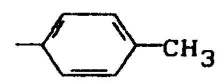 | 155-169° C | L |
| 32 | 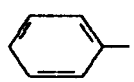 | H | H | $-CH-CH_2-CH_3$<br>$\vert$<br>$CH_3$ | H | H | $CH_3$ | 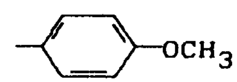 | 154-161° C | L |

**Tabelle 1** Fortsetzung

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 33 | 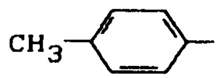 | H | H | $-CH(CH_3)_2$ | H | H | $CH_3$ | 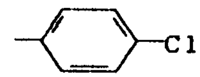 | 126-127° C | L |
| 34 | 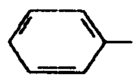 | H | H | $-CH(CH_3)-CH_2-CH_3$ | H | H | $-CH_2CH_3$ | 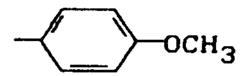 | 145-150° C | L |
| 35 | 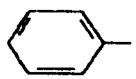 | H | H | $-CH(CH_3)_2$ | H | H | $-CH_2CH_3$ | 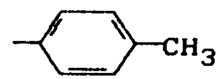 | 150-156° C | L |
| 36 | 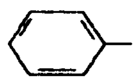 | H | H | $-CH(CH_3)_2$ | H | H | $-CH_2CH_3$ | 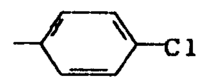 | 146-149° C | L |
| 37 | 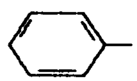 | H | H | $-CH(CH_3)_2$ | H | H | $CH_3$ | 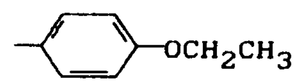 | 149-150° C | L |
| 38 | 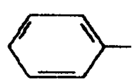 | H | H | $-CH(CH_3)_2$ | H | H | $CH_3$ | 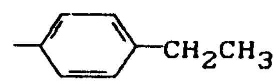 | 158-160° C | L |

Tabelle 1 Fortsetzung

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 39 | 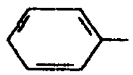 | H | H | $-CH(CH_3)_2$ | H | H | $CH_3$ | 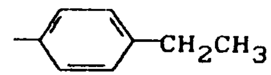 | 112-135° C | D/L |
| 40 | 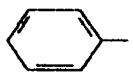 | H | H | $-CH(CH_3)_2$ | H | H | $CH_3$ | 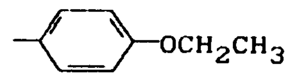 | 121-137° C | D/L |
| 41 | 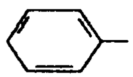 | H | H | $-CH(CH_3)_2$ | H | H | $-CH_2CH_3$ | 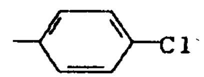 | 155-172° C | D/L |
| 42 | 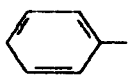 | H | H | $-CH(CH_3)_2$ | H | H | $-CH_2CH_3$ | 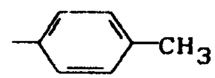 | 138-140° C | D/L |
| 43 | 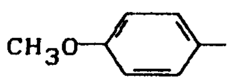 | H | H | $-CH(CH_3)_2$ | H | H | $CH_3$ | 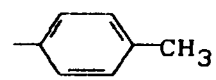 | 149-159° C | D/L |
| 44 | 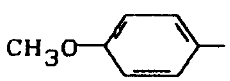 | H | H | $-CH(CH_3)_2$ | H | H | $CH_3$ | 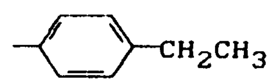 | 148-155° C | D/L |

<u>Tabelle 1</u> Fortsetzung

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 45 | 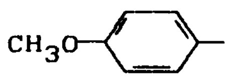 | H | H | $-CH(CH_3)_2$ | H | H | $-CH_2CH_3$ | 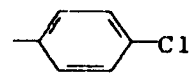 | 149-163° C | D/L |
| 46 | 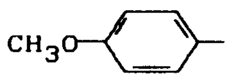 | H | H | $-CH(CH_3)_2$ | H | H | $-CH_2CH_3$ | 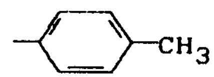 | 146-158° C | D/L |
| 47 | 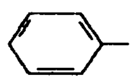 | H | H | $-CH(CH_3)_2$ | H | H | $CH_3$ | 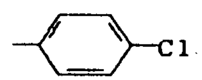 | 151-153° C | D/L |
| 48 | 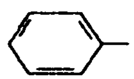 | H | H | $-CH(CH_3)_2$ | H | H | $CH_3$ | 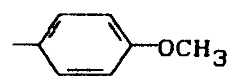 | 140-143° C | D/L |
| 49 | 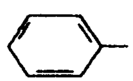 | H | H | $-CH(CH_3)_2$ | H | H | $CH_3$ | $-(CH_2)_2$ 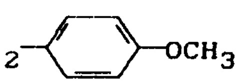 | 143-151° C | D/L |

**Tabelle 1** Fortsetzung

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 50 | 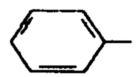 | H | H | $-CH(CH_3)_2$ | H | H | $CH_3$ | 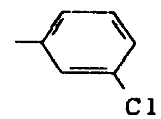 | 138-148° C | L |
| 51 | 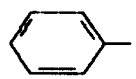 | H | H | $-CH(CH_3)_2$ | H | H | $CH_3$ | 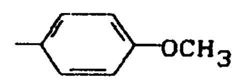 | 145-153° C | L |
| 52 | 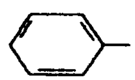 | H | H | $-CH(CH_3)_2$ | H | H | $CH_3$ | $-(CH_2)_2$ 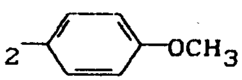 | 120-129° C | L |
| 53 | 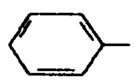 | H | H | $-CH(CH_3)_2$ | H | H | $CH_3$ | 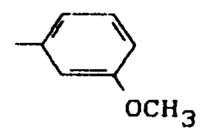 | 152-158° C | L |
| 54 | 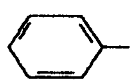 | H | H | $-CH(CH_3)_2$ | H | H | $CH_3$ | 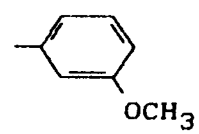 | 133-138° C | D/L |

Tabelle 1 Fortsetzung

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 55 | 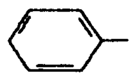 | H | H | $-CH(CH_3)_2$ | H | H | $CH_3$ | 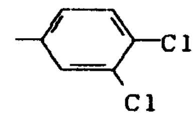 | 168-177° C | D/L |
| 56 | 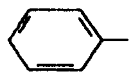 | H | H | $-CH(CH_3)_2$ | H | H | $CH_3$ | 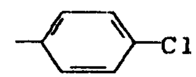 | 166-169° C R-Amin | L |
| 57 | 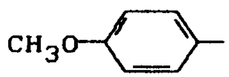 | H | H | $-CH(CH_3)_2$ | H | H | $CH_3$ | 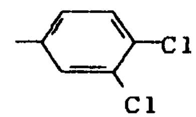 | 153-165° C | D/L |
| 58 | 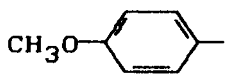 | H | H | $-CH(CH_3)_2$ | H | H | $CH_3$ | 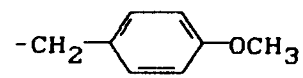 | 143-147° C | D/L |
| 59 | 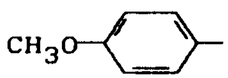 | H | H | $-CH(CH_3)_2$ | H | H | $CH_3$ | 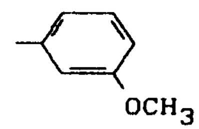 | 129-130° C | D/L |
| 60 | 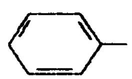 | H | H | $-CH(CH_3)_2$ | H | H | $CH_3$ | 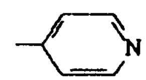 | 152-155° C | D/L |

**Tabelle 1** Fortsetzung

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 61 | 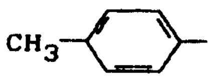 | H | H | $-CH(CH_3)_2$ | H | H | $CH_3$ | 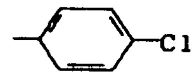 | 138° C (R+)-Amine | L |
| 62 | 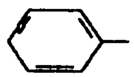 | H | $-CH_2-CH_2-CH_2-CH_2-$ | | H | H | $CH_3$ | 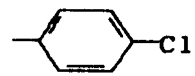 | 158° C | |
| 63 | 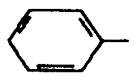 | H | $-CH_2-CH_2-CH_2-CH_2-$ | | H | H | $CH_3$ | 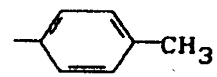 | 164° C | |
| 64 | 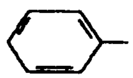 | H | $-CH_2-CH_2-CH_2-CH_2-$ | | H | H | $CH_3$ | 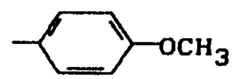 | 178° C | |
| 65 | 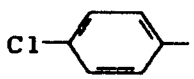 | H | H | $-CH(CH_3)_2$ | H | H | $CH_3$ | 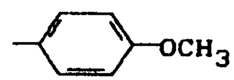 | 168-170° C | L |
| 66 | 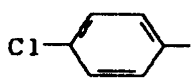 | H | H | $-CH(CH_3)_2$ | H | H | $CH_3$ | 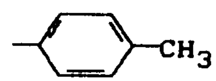 | 152-° C | L |

<u>Tabelle 1</u> Fortsetzung

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 67 | 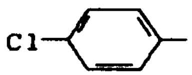 | H | H | $-CH(CH_3)_2$ | H | H | $CH_3$ | 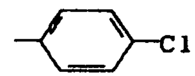 | 179° C (R+)-Amine | L |
| 68 | 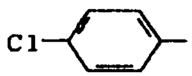 | H | H | $-CH(CH_3)_2)-$ | H | H | $CH_3$ | 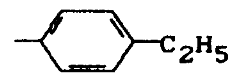 | 159° C | L |
| 69 | 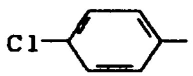 | H | H | $-CH(CH_3)_2$ | H | H | $CH_3$ | 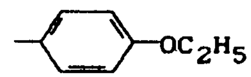 | 164° C | L |
| 70 | 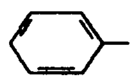 | H | H | $-C_4H_9n$ | H | H | $CH_3$ | 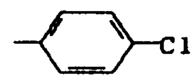 | 133° C | L |
| 71 | 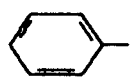 | H | H | $-C_4H_9n$ | H | H | $CH_3$ | 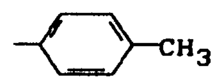 | 120° C | L |
| 72 | 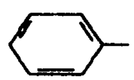 | H | H | $-C_4H_9n$ | H | H | $CH_3$ | 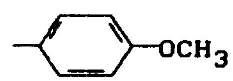 | 141° C | L |

Tabelle 1 Fortsetzung

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 73 | 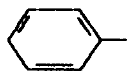 | H | H | $-C_4H_9b$ | H | H | $CH_3$ | 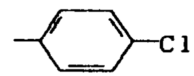 | 132° C | L |
| 74 | 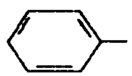 | H | H | $-C_4H_9n$ | H | H | $CH_3$ | 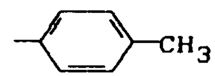 | 140° C | L |
| 75 | 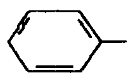 | H | H | $-C_4H_9n$ | H | H | $CH_3$ | 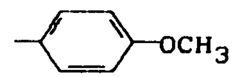 | 136-139° C | L |
| 76 | 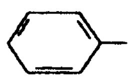 | H | $-CH_2-CH_2-$ | | H | H | $CH_3$ | 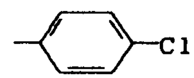 | 145° C | |
| 77 | 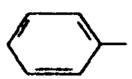 | H | $-CH_2-CH_2-$ | | H | H | $CH_3$ | 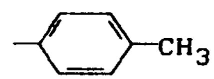 | 146° C | |
| 78 | 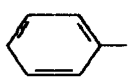 | H | $-CH_2-CH_2-$ | | H | H | $CH_3$ | 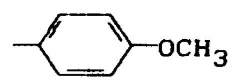 | 133° C | L |

**Tabelle 1** Fortsetzung

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 79 | 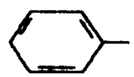 | H | H | $-C_4H_9t$ | H | H | $CH_3$ | 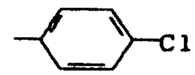 | 154° C | |
| 80 | 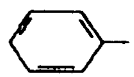 | H | H | $-C_4H_9t$ | H | H | $CH_3$ | 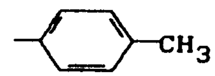 | 155° C | |
| 81 | 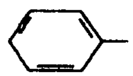 | H | H | $-C_4H_9t$ | H | H | $CH_3$ | 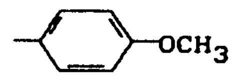 | 152° C | D/L |
| 82 | 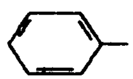 | H | H | $-C_2H_5$ | H | H | $CH_3$ | 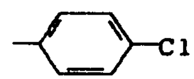 | 135° C | L |
| 83 | 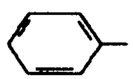 | H | H | $-C_2H_5$ | H | H | $CH_3$ | 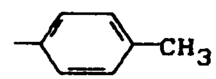 | 143° C | L |
| 84 | 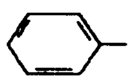 | H | H | $-C_2H_5$ | H | H | $CH_3$ | 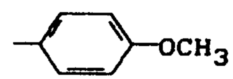 | 140° C | L |

Tabelle 1 Fortsetzung

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 85 | 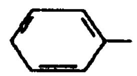 | H | H | $-CH-(CH_3)_2)$ | H | H | $CH_3$ | 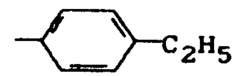 | 156-158° C (R+)-Amin | L |
| 86 | 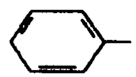 | H | H | $-CH-(CH_3)_2$ | H | H | $CH_3$ | 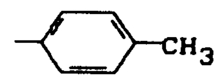 | 168-170° C (R+)-Amin | L |
| 87 | 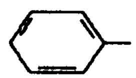 | H | H | $-CH-(CH_3)_2$ | H | H | $CH_3$ | 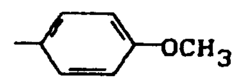 | 166-168° C (R+)-Amin | L |
| 88 | 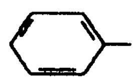 | H | H | $-CH-(CH_3)_2$ | H | H | $CH_3$ | 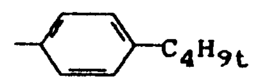 | 97° C | L |
| 89 | 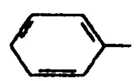 | H | H | $-CH-(CH_3)_2$ | H | H | $CH_3$ | 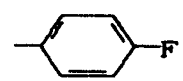 | 151° C | L |
| 90 | 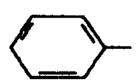 | H | H | $-CH-(CH_3)_2$ | H | H | $CH_3$ | 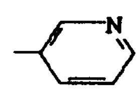 | | L |

Tabelle 1 Fortsetzung

| Nr. | Ar | $R^2$ | $R^3$ | $R^4$ | $R^1$ | $R^5$ | $R^6$ | Ar' | phys. Konstante | * |
|---|---|---|---|---|---|---|---|---|---|---|
| 91 | 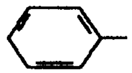 | H | H | $-CH-(CH_3)_2$) | H | H | $CH_3$ | 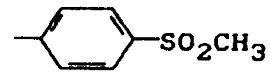 | 136° C | L |
| 92 | 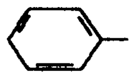 | H | H | $-CH-(CH_3)_2$ | H | H | $CH_3$ | 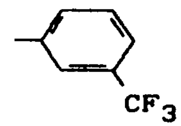 | 148° C | L |
| 93 | 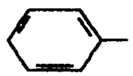 | H | H | $-CH-(CH_3)_2$ | H | H | $CH_3$ | 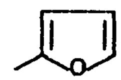 | 142° C | L |
| 94 | 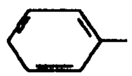 | H | H | $-CH-(CH_3)_2$ | H | H | $CH_3$ | 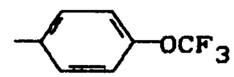 | 162° C | L |
| 95 | 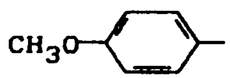 | H | H | $-CH-(CH_3)_2$ | H | H | $CH_3$ | 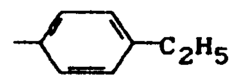 | 159° C (R+)-Amin | L |
| 96 | 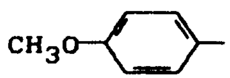 | H | H | $-CH-(CH_3)_2$ | H | H | $CH_3$ | 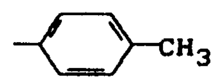 | 181° C (R+)-Amin | L |
| 97 | 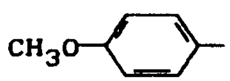 | H | H | $-CH-(CH_3)_2$ | H | H | $CH_3$ | 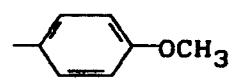 | 174° C (R+)-Amin | L |

Beispiel A

Plasmopara-Test (Reben) / protektiv

Lösungsmittel: 4,7 Gewichtsteile Aceton
Emulgator: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 °C und 100 % relativer Luftfeuchtigkeit. Anschließend werden die Pflanzen 5 Tage im Gewächshaus bei 22 °C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

So zeigen in diesem Test z.B. die Verbindungen gemäß der Herstellungsbeispiele (1), (2), (4), (6), (8), (9), (10), (11), (12), (13) und (14) bei einer beispielhaften Wirkstoffkonzentration von 5 ppm einen ausgezeichneten Wirkungsgrad.

Beispiel B

Phytophthora-Test (Tomate) /protektiv

Lösungsmittel: 4,7 Gewichtsteile Aceton
Emulgator: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20 °C aufgestellt.

3 Tage nach der Inokulation erfolgt die Auswertung.

Z. B. zeigen die Verbindungen gemäß der Herstellungsbeispiele (1), (4), (7) und (8) bis (16) bei einer beispielhaften Wirkstoffkonzentration von 5 ppm einen ausgezeichneten Wirkungsgrad.

**Patentansprüche**

**Patentansprüche für folgende Vertragsstaaten : BE, CH, DE, DK, FR, GB, GR, IT, LI, NL**

**1.** Aminosäureamid-Derivate der allgemeinen Formel (1)

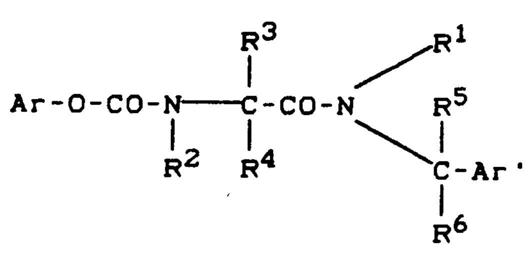

(I),

in welcher

Ar und Ar' gleich oder verschieden sind und für unsubstituiertes oder substituiertes Aryl, unsubstituiertes oder substituiertes Aralkyl, unsubstituiertes oder substituiertes Heteroaryl und unsubstituiertes oder substituiertes Heteroarylalkyl stehen,

| | |
|---|---|
| $R^1$, $R^2$, $R^3$, $R^4$, $R^5$ und $R^6$ | gleich oder verschieden sind und für Wasserstoff oder Alkyl stehen oder |
| $R^3$ und $R^4$ | zusammen mit dem Kohlenstoffatom, an das sie gebunden sind, einen Cycloalkylring bilden. |

**2.** Aminosäureamid-Derivate der Formel (I) gemäß Anspruch 1, in welcher

| | |
|---|---|
| $R^1$, $R^2$, $R^3$, $R^4$, $R^5$ und $R^6$ | gleich oder verschieden sind und für Wasserstoff oder geradkettiges oder verzweigtes Alkyl mit 1 bis 6 Kohlenstoffatomen stehen oder |
| $R^3$ und $R^4$ | zusammen mit dem Kohlenstoffatom, an das sie gebunden sind, einen Cycloalkylring mit 3 bis 7 Kohlenstoffatomen bilden und |
| Ar und Ar′ | gleich oder verschieden sind und für jeweils unsubstituiertes oder substituiertes Phenyl, Furyl und Pyridyl oder unsubstituiertes oder im Phenylteil substituiertes Phenylalkyl mit 1 bis 4 Kohlenstoffatomen im geradkettigen oder verzweigten Alkylteil stehen, wobei als Substituenten im Phenylteil jeweils in Frage kommen: Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 9 Halogenatomen, wobei die Halogenatome gleich oder verschieden sind; Hydroxy; Halogen; Cyano; Nitro; Dialkylamino mit 1 bis 4 Kohlenstoffatomen je Alkylgruppe; Carboxy; Alkylalkoxy mit 1 bis 4 Kohlenstoffatomen in jedem Alkylteil; Carbonylalkoxy mit 1 bis 4 Kohlenstoffatomen im Alkylteil; Carbonylalkyl mit 1 bis 4 Kohlenstoffatomen in Alkylteil; Formyl; Carbonylaryloxy mit 5 bis 10 Kohlenstoffatomen im Arylteil; Carbonylaryl mit 6 bis 10 Kohlenstoffatomen im Arylteil; Oxycarbonylalkyl mit 1 bis 4 Kohlenstoffatomen im Alkylteil; Oxycarbonylaryl mit 6 bis 10 Kohlenstoffatomen im Arylteil; Carbonylamino, Carbonylaminoalkyl, Carbonylaminodialkyl, Aminocarbonyl, Alkylaminocarbonyl, Aminocarbonylalkyl und Alkylaminocarbonylalkyl mit jeweils 1 bis 4 Kohlenstoffatomen im Alkylteil; Sulfonamido; Sulfonalkyl; Sulfonylalkyl und Sulfonylalkoxy mit jeweils 1 bis 4 Kohlenstoffatomen; jeweils unsubstituiertes oder durch Halogen substituiertes Phenyl oder Phenoxy. |

**3.** Aminosäureamid-Derivate der Formel (I) gemäß Anspruch 1, in welcher

| | |
|---|---|
| $R^1$ | für Wasserstoff steht und |
| $R^2$, $R^3$ und $R^4$ | gleich oder verschieden sind und für Wasserstoff oder geradkettiges oder verzweigtes Alkyl mit 1 bis 5 Kohlenstoffatomen stehen oder |
| $R^3$ und $R^4$ | zusammen mit dem Kohlenstoffatom, an das sie gebunden sind, einen Cycloalkylring mit 3 bis 6 Kohlenstoffatomen bilden und |
| $R^5$ und $R^6$ | gleich oder verschieden sind und für Wasserstoff, Methyl oder Ethyl stehen und |
| Ar | für unsubstituiertes oder einfach bis dreifach, gleich oder verschieden substituiertes Phenyl steht, wobei die folgenden Substituenten in Frage kommen: Alkyl, Alkoxy und Alkylthio mit jeweils 1 oder 2 Kohlenstoffatomen; Halogenalkyl, Halogenalkoxy und Halogenalkylthio mit 1 oder 2 Kohlenstoffatomen und 1 bis 5 Halogenatomen, wobei die Halogenatome gleich oder verschieden sind; Hydroxy; Fluor, Chlor, Brom und Jod; Cyano; Nitro; Dialkylamino mit 1 oder 2 Kohlenstoffatomen je Alkylgruppe; Carboxy; Alkylalkoxy mit 1 oder 2 Kohlenstoffatomen in jedem Alkylteil; Carbonylalkoxy mit 1 oder 2 Kohlenstoffatomen im Alkylteil; Carbonylalkyl mit 1 oder 2 Kohlenstoffatomen im Alkylteil; Formyl; Carbonylphenoxy; Benzoyl; Oxycarbonylalkyl mit 1 oder 2 Kohlenstoffatomen; Benzoyloxy; Carbonylamino, Carbonylaminoalkyl,Carbonylaminodialkyl, Aminocarbonyl, Alkylaminocarbonyl, Aminocarbonylalkyl und Alkylaminocarbonylalkyl mit jeweils 1 oder 2 Kohlenstoffatomen im Alkylteil; Sulfonamido; Sulfonalkyl, Sulfonylalkyl und Sulfonylalkoxy mit jeweils 1 oder 2 Kohlenstoffatomen; jeweils unsubstituiertes oder durch Fluor, Chlor oder Brom substituiertes Phenyl oder Phenoxy und |
| Ar′ | für jeweils unsubsituiertes oder einfach bis dreifach, gleich oder verschieden substituiertes Phenyl, Furyl oder Pyridyl oder für unsubstituiertes oder im Phenylteil einfach bis dreifach, gleich oder verschieden substituiertes Phenylalkyl mit 1 |

oder 2 Kohlenstoffatomen im Alkylteil steht, wobei als Phenylsubstituenten jeweils die oben genannten Phenylsubstituenten in Frage kommen.

**4.** Aminosäureamid-Derivate der Formel (I), gemäß Anspruch 1, in welcher

| | |
|---|---|
| $R^1$ | für Wasserstoff steht und |
| $R^2$ | für Wasserstoff oder Methyl steht und |
| $R^3$ und $R^4$ | gleich oder verschieden sind und für Wasserstoff, Methyl, Ethyl, n- oder i-Propyl, n-, i-, s-oder t-Butyl oder 3-Pentyl stehen oder |
| $R^3$ und $R^4$ | zusammen mit dem Kohlenstoffatom, an das sie gebunden sind, einen Cyclopropyl-, Cyclopentyl- oder Cyclohexylring bilden und |
| $R^5$ | für Wasserstoff oder Methyl steht und |
| $R^6$ | für Wasserstoff, Methyl oder Ethyl steht und |
| Ar | für unsubstituiertes oder einfach bis zweifach, gleich oder verschieden substituiertes Phenyl steht, wobei die folgenden Substituenten in Frage kommen: Methyl, Ethyl, n- und i-Propyl, n-, i-, s- und t-Butyl, Methoxy, Ethoxy, i- und n-Propoxy, n-, i-, s- und t-Butoxy, Trifluormethyl, Difluormethyl, Pentafluorethyl, Tetrafluorethyl, Trifluorchlorethyl, Trifluorethyl, Trifluorethoxy, Difluormethoxy, Pentafluorethoxy, Tetrafluorethoxy, Trifluorchlorethoxy, Trifluormethoxy und Trifluormethylthio; Chlor, Brom, Fluor, Nitro und Cyano. |
| Ar' | für jeweils unsubstituiertes oder im Phenylteil einfach bis zweifach, gleich oder verschieden substituiertes Benzyl, 1,2-Phenethyl oder Phenyl steht, wobei als Phenylsubstituenten jeweils die oben genannten Phenylsubstituenten in Frage kommen. |

**5.** Aminosäureamid-Derivate der allgemeinen Formel (I) gemäß Anspruch 1, in welcher

| | |
|---|---|
| $R^1$, $R^2$, $R^5$, $R^6$, Ar und Ar' | die in einem der Ansprüche 1 bis 4 angegebenenen Bedeutungen haben und |
| $R^3$ | für Wasserstoff steht und |
| $R^4$ | für Methyl, Ethyl, n-Propyl, t-Butyl, i-Propyl, i-Butyl, s-Butyl oder 3-Pentyl steht oder |
| $R^3$ und $R^4$ | zusammen mit dem Kohlenstoffatom, an das sie gebunden sind, einen Cycloalkylring mit 3 bis 6 Kohlenstoffatomen bilden. |

**6.** Verfahren zur Herstellung von Aminosäureamid-Derivaten der allgemeinen Formel (I)

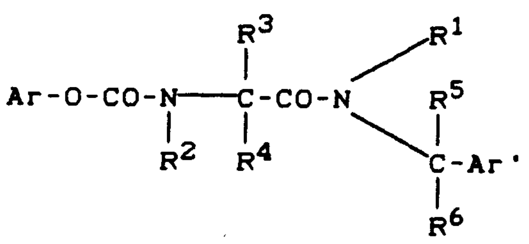

(I),

in welcher

| | |
|---|---|
| Ar und Ar' | gleich oder verschieden sind und für jeweils unsubstituiertes oder substituiertes Aryl, Aralkyl, Heteroaryl oder Heteroarylalkyl stehen, |
| $R^1$, $R^2$, $R^3$, $R^4$, $R^5$ und $R^6$ | gleich oder verschieden sind und für Wasserstoff oder Alkyl stehen oder |
| $R^3$ und $R^4$ | zusammen mit dem Kohlenstoffatom, an das sie gebunden sind, einen Cycloalkylring bilden, |

dadurch gekennzeichnet, daß man
eine substituierte Aminosäure der Formel (II)

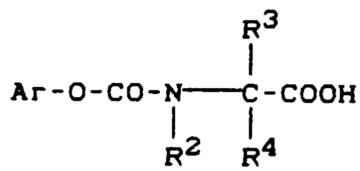

(II),

in welcher

Ar, $R^2$, $R^3$ und $R^4$ die oben angegebene Bedeutung haben, bzw. deren carboxy-aktivierte Derivate,

gegebenenfalls in Gegenwart eines Katalysators, gegebenenfalls in Gegenwart eines Säurebindemittels und gegebenenfalls in Gegenwart eines Verdünnungsmittels mit einem Amin der Formel (III)

$HNR^1-CR^5R^6Ar'$ (III),

in welcher

Ar', $R^1$, $R^5$ und $R^6$ die oben angegebene Bedeutung haben, umsetzt.

**7.** Schädlingsbekämpfungsmittel, gekennzeichnet durch einen Gehalt an mindestens einem Aminosäureamid-Derivat der Formel (I) nach den Ansprüchen 1 bis 6.

**8.** Verwendung von Aminosäureamid-Derivaten der Formel (I) nach den Ansprüchen 1 bis 6 zur Bekämpfung von Schädlingen.

**9.** Verfahren zur Bekämpfung von Schädlingen, dadurch gekennzeichnet, daß man Aminosäureamid-Derivate der Formel (I) nach den Ansprüchen 1 bis 6 auf Schädlinge und/oder ihren Lebensraum einwirken läßt.

**10.** Verfahren zur Herstellung von Schädlingsbekämpfungsmitteln, dadurch gekennzeichnet, daß man Aminosäureamid-Derivate der Formel (I) nach den Ansprüchen 1 bis 6 mit Streckmitteln und/oder oberflächenaktiven Mittel vermischt.

**Patentansprüche für folgenden Vertragsstaat : ES**

**1.** Verfahren zur Herstellung von Aminosäureamid-Derivaten der allgemeinen Formel (I)

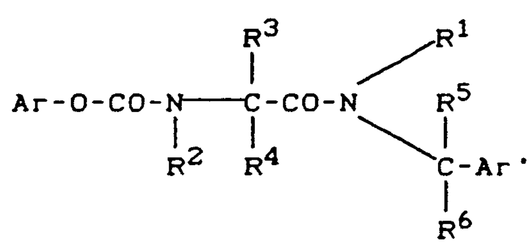

(I),

in welcher

| | |
|---|---|
| Ar und Ar' | gleich oder verschieden sind und für jeweils unsubstituiertes oder substituiertes Aryl, Aralkyl, Heteroaryl oder Heteroarylalkyl stehen, |
| $R^1$, $R^2$, $R^3$, $R^4$, $R^5$ und $R^6$ | gleich oder verschieden sind und für Wasserstoff oder Alkyl stehen oder |
| $R^3$ und $R^4$ | zusammen mit dem Kohlenstoffatom, an das sie gebunden sind, einen Cycloalkylring bilden, |

dadurch gekennzeichnet, daß man

eine substituierte Aminosäure der Formel (II)

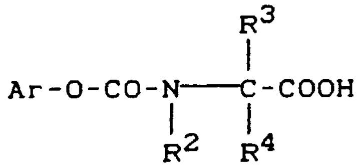 (II),

in welcher

Ar, $R^2$, $R^3$ und $R^4$ die oben angegebene Bedeutung haben, bzw. deren carboxy-aktivierte Derivate,

gegebenenfalls in Gegenwart eines Katalysators, gegebenenfalls in Gegenwart eines Säurebindemittels und gegebenenfalls in Gegenwart eines Verdünnungsmittels
mit einem Amin der Formel (III)

$HNR^1-CR^5R^6Ar'$ (III),

in welcher
Ar', $R^1$, $R^5$ und $R^6$ die oben angegebene Bedeutung haben, umsetzt.

**2.** Verfahren gemäß Anspruch 1, in welcher

| | |
|---|---|
| $R^1$, $R^2$, $R^3$, $R^4$, $R^5$ und $R^6$ | gleich oder verschieden sind und für Wasserstoff oder geradkettiges oder verzweigtes Alkyl mit 1 bis 6 Kohlenstoffatomen stehen oder |
| $R^3$ und $R^4$ | zusammen mit dem Kohlenstoffatom, an das sie gebunden sind, einen Cycloalkylring mit 3 bis 7 Kohlenstoffatomen bilden und |
| Ar und Ar' | gleich oder verschieden sind und für jeweils unsubstituiertes oder substituiertes Phenyl, Furyl und Pyridyl oder unsubstituiertes oder im Phenylteil substituiertes Phenylalkyl mit 1 bis 4 Kohlenstoffatomen im geradkettigen oder verzweigten Alkylteil stehen, wobei als Substituenten im Phenylteil jeweils in Frage kommen: Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 9 Halogenatomen, wobei die Halogenatome gleich oder verschieden sind; Hydroxy; Halogen; Cyano; Nitro; Dialkylamino mit 1 bis 4 Kohlenstoffatomen je Alkylgruppe; Carboxy; Alkylalkoxy mit 1 bis 4 Kohlenstoffatomen in jedem Alkylteil; Carbonylalkoxy mit 1 bis 4 Kohlenstoffatomen im Alkylteil; Carbonylalkyl mit 1 bis 4 Kohlenstoffatomen in Alkylteil; Formyl; Carbonylaryloxy mit 5 bis 10 Kohlenstoffatomen im Arylteil; Carbonylaryl mit 6 bis 10 Kohlenstoffatomen im Arylteil; Oxycarbonylalkyl mit 1 bis 4 Kohlenstoffatomen im Alkylteil; Oxycarbonylaryl mit 6 bis 10 Kohlenstoffatomen im Arylteil; Carbonylamino, Carbonylaminoalkyl, Carbonylaminodialkyl, Aminocarbonyl, Alkylaminocarbonyl, Aminocarbonylalkyl und Alkylaminocarbonylalkyl mit jeweils 1 bis 4 Kohlenstoffatomen im Alkylteil; Sulfonamido; Sulfonalkyl; Sulfonylalkyl und Sulfonylalkoxy mit jeweils 1 bis 4 Kohlenstoffatomen; jeweils unsubstituiertes oder durch Halogen substituiertes Phenyl oder Phenoxy. |

**3.** Verfahren gemäß Anspruch 1, in welcher

| | |
|---|---|
| $R^1$ | für Wasserstoff steht und |
| $R^2$, $R^3$ und $R^4$ | gleich oder verschieden sind und für Wasserstoff oder geradkettiges oder verzweigtes Alkyl mit 1 bis 5 Kohlenstoffatomen stehen oder |
| $R^3$ und $R^4$ | zusammen mit dem Kohlenstoffatom, an das sie gebunden sind, einen Cycloalkylring mit 3 bis 6 Kohlenstoffatomen bilden und |
| $R^5$ und $R^6$ | gleich oder verschieden sind und für Wasserstoff, Methyl oder Ethyl stehen und |
| Ar | für unsubstituiertes oder einfach bis dreifach, gleich oder verschieden substituiertes Phenyl steht, wobei die folgenden Substituenten in Frage kommen: Alkyl, Alkoxy und Alkylthio mit jeweils 1 oder 2 Kohlenstoffatomen; Halogenalkyl, Halogenalkoxy und Halogenalkylthio mit 1 oder 2 Kohlenstoffatomen und 1 bis 5 |

Halogenatomen, wobei die Halogenatome gleich oder verschieden sind; Hydroxy; Fluor, Chlor, Brom und Jod; Cyano; Nitro; Dialkylamino mit 1 oder 2 Kohlenstoffatomen je Alkylgruppe; Carboxy; Alkylalkoxy mit 1 oder 2 Kohlenstoffatomen in jedem Alkylteil; Carbonylalkoxy mit 1 oder 2 Kohlenstoffatomen im Alkylteil; Carbonylalkyl mit 1 oder 2 Kohlenstoffatomen im Alkylteil; Formyl; Carbonylphenoxy; Benzoyl; Oxycarbonylalkyl mit 1 oder 2 Kohlenstoffatomen; Benzoyloxy; Carbonylamino, Carbonylaminoalkyl,Carbonylaminodialkyl, Aminocarbonyl, Alkylaminocarbonyl, Aminocarbonylalkyl und Alkylaminocarbonylalkyl mit jeweils 1 oder 2 Kohlenstoffatomen im Alkylteil; Sulfonamido; Sulfonalkyl, Sulfonylalkyl und Sulfonylalkoxy mit jeweils 1 oder 2 Kohlenstoffatomen; jeweils unsubstituiertes oder durch Fluor, Chlor oder Brom substituiertes Phenyl oder Phenoxy und

Ar' für jeweils unsubsituiertes oder einfach bis dreifach, gleich oder verschieden substituiertes Phenyl, Furyl oder Pyridyl oder für unsubstituiertes oder im Phenylteil einfach bis dreifach, gleich oder verschieden substituiertes Phenylalkyl mit 1 oder 2 Kohlenstoffatomen im Alkylteil steht, wobei als Phenylsubstituenten jeweils die oben genannten Phenylsubstituenten in Frage kommen.

**4.** Verfahren, gemäß Anspruch 1, in welcher

$R^1$ für Wasserstoff steht und

$R^2$ für Wasserstoff oder Methyl steht und

$R^3$ und $R^4$ gleich oder verschieden sind und für Wasserstoff, Methyl, Ethyl, n- oder i-Propyl, n-, i-, s-oder t-Butyl oder 3-Pentyl stehen oder

$R^3$ und $R^4$ zusammen mit dem Kohlenstoffatom, an das sie gebunden sind, einen Cyclopropyl-, Cyclopentyl- oder Cyclohexylring bilden und

$R^5$ für Wasserstoff oder Methyl steht und

$R^6$ für Wasserstoff, Methyl oder Ethyl steht und

Ar für unsubstituiertes oder einfach bis zweifach, gleich oder verschieden substituiertes Phenyl steht, wobei die folgenden Substituenten in Frage kommen: Methyl, Ethyl, n- und i-Propyl, n-, i-, s- und t-Butyl, Methoxy, Ethoxy, i- und n-Propoxy, n-, i-, s- und t-Butoxy, Trifluormethyl, Difluormethyl, Pentafluorethyl, Tetrafluorethyl, Trifluorchlorethyl, Trifluorethyl, Trifluorethoxy, Difluormethoxy, Pentafluorethoxy, Tetrafluorethoxy, Trifluorchlorethoxy, Trifluormethoxy und Trifluormethylthio; Chlor, Brom, Fluor, Nitro und Cyano.

Ar' für jeweils unsubstituiertes oder im Phenylteil einfach bis zweifach, gleich oder verschieden substituiertes Benzyl, 1,2-Phenethyl oder Phenyl steht, wobei als Phenylsubstituenten jeweils die oben genannten Phenylsubstituenten in Frage kommen.

**5.** Verfahren gemäß Anspruch 1, in welcher

$R^1$, $R^2$, $R^5$, $R^6$, Ar und Ar' die in einem der Ansprüche 1 bis 4 angegebenenen Bedeutungen haben und

$R^3$ für Wasserstoff steht und

$R^4$ für Methyl, Ethyl, n-Propyl, t-Butyl, i-Propyl, i-Butyl, s-Butyl oder 3-Pentyl steht oder

$R^3$ und $R^4$ zusammen mit dem Kohlenstoffatom, an das sie gebunden sind, einen Cycloalkylring mit 3 bis 6 Kohlenstoffatomen bilden.

**6.** Schädlingsbekämpfungsmittel, gekennzeichnet durch einen Gehalt an mindestens einem Aminosäureamid-Derivat der Formel (I) nach den Ansprüchen 1 bis 5.

**7.** Verwendung von Aminosäureamid-Derivaten der Formel (I) nach den Ansprüchen 1 bis 5 zur Bekämpfung von Schädlingen.

**8.** Verfahren zur Bekämpfung von Schädlingen, dadurch gekennzeichnet, daß man Aminosäureamid-Derivate der Formel (I) nach den Ansprüchen 1 bis 5 auf Schädlinge und/oder ihren Lebensraum einwirken läßt.

**9.** Verfahren zur Herstellung von Schädlingsbekämpfungsmitteln, dadurch gekennzeichnet, daß man Aminosäureamid-Derivate der Formel (I) nach den Ansprüchen 1 bis 5 mit Streckmitteln und/oder oberflächenaktiven Mittel vermischt.

## Claims

**Claims for the following Contracting States : BE, CH, DE, DK, FR, GB, GR, IT, LI, NL**

**1.** Amino acid amide derivatives of the general formula (I)

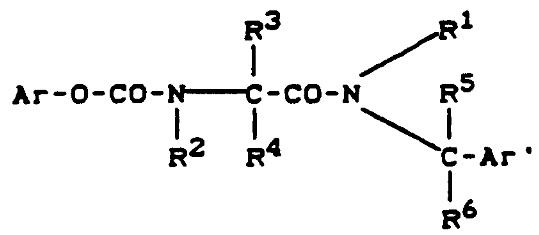

(I)

in which

| | |
|---|---|
| Ar and Ar' | are identical or different and represent unsubstituted or substituted aryl, unsubstituted or substituted aralkyl, unsubstituted or substituted heteroaryl and unsubstituted or substituted heteroarylalkyl, |
| $R^1$, $R^2$, $R^3$, $R^4$, $R^5$ and $R^6$ | are identical or different and represent hydrogen or alkyl or |
| $R^3$ and $R^4$, | together with the carbon atom to which they are bonded, form a cycloalkyl ring. |

**2.** Amino acid amide derivatives of the formula (I) according to Claim 1, in which

| | |
|---|---|
| $R^1$, $R^2$, $R^3$, $R^4$, $R^5$ and $R^6$ | are identical or different and represent hydrogen or straight-chain or branched alkyl having 1 to 6 carbon atoms or |
| $R^3$ and $R^4$, | together with the carbon atom to which they are bonded, form a cycloalkyl ring having 3 to 7 carbon atoms and |
| Ar and Ar' | are identical or different and represent phenyl, furyl and pyridyl, in each case unsubstituted or substituted, or phenylalkyl which is unsubstituted or substituted in the phenyl moiety and which has 1 to 4 carbon atoms in the straight-chain or branched alkyl moiety, suitable substituents in the phenyl moiety in each case being: alkyl, alkoxy and alkylthio each having 1 to 4 carbon atoms; halogenoalkyl, halogenoalkoxy and halogenoalkylthio each having 1 to 4 carbon atoms and 1 to 9 halogen atoms, the halogen atoms being identical or different; hydroxyl; halogen; cyano; nitro; dialkylamino having 1 to 4 carbon atoms per alkyl group; carboxyl; alkylalkoxy having 1 to 4 carbon atoms in each alkyl moiety; carbonylalkoxy having 1 to 4 carbon atoms in the alkyl moiety; carbonylalkyl having 1 to 4 carbon atoms in the alkyl moiety; formyl; carbonylaryloxy having 5 to 10 carbon atoms in the aryl moiety; carbonylaryl having 6 to 10 carbon atoms in the aryl moiety; oxycarbonylalkyl having 1 to 4 carbon atoms in the alkyl moiety; oxycarbonylaryl having 6 to 10 carbon atoms in the aryl moiety; carbonylamino, carbonylaminoalkyl, carbonylaminodialkyl, aminocarbonyl, alkylaminocarbonyl, aminocarbonylalkyl and alkylaminocarbonylalkyl each having 1 to 4 carbon atoms in the alkyl moiety; sulphonamido; sulphonalkyl; sulphonylalkyl and sulphonylalkoxy each having 1 to 4 carbon atoms; phenyl or phenoxy, in each case unsubstituted or substituted by halogen. |

**3.** Amino acid amide derivatives of the formula (I) according to Claim 1, in which

| | |
|---|---|
| $R^1$ | represents hydrogen and |
| $R^2$, $R^3$ and $R^4$ | are identical or different and represent hydrogen or straight-chain or branched alkyl having 1 to 5 carbon atoms or |
| $R^3$ and $R^4$, | together with the carbon atom to which they are bonded, form a cycloalkyl ring having 3 to 6 carbon atoms and |
| $R^5$ and $R^6$ | are identical or different and represent hydrogen, methyl or ethyl and |

Ar represents phenyl which is unsubstituted or monosubstituted to trisubstituted by identical or different substituents, suitable substituents being the following: alkyl, alkoxy and alkylthio each having 1 or 2 carbon atoms; halogenoalkyl, halogenoalkoxy and halogenoalkylthio having 1 or 2 carbon atoms and 1 to 5 halogen atoms, the halogen atoms being identical or different; hydroxyl; fluorine, chlorine, bromine and iodine; cyano; nitro; dialkylamino having 1 or 2 carbon atoms per alkyl group; carboxyl; alkylalkoxy having 1 or 2 carbon atoms in each alkyl moiety; carbonylalkoxy having 1 or 2 carbon atoms in the alkyl moiety; carbonylalkyl having 1 or 2 carbon atoms in the alkyl moiety; formyl; carbonylphenoxy; benzoyl; oxycarbonylalkyl having 1 or 2 carbon atoms; benzoyloxy; carbonylamino, carbonylaminoalkyl, carbonylaminodialkyl, aminocarbonyl, alkylaminocarbony-l, aminocarbonylalkyl and alkylaminocarbonylalkyl each having 1 or 2 carbon atoms in the alkyl moiety; sulphonamido; sulphonalkyl, sulphonylalkyl and sulphonylalkoxy each having 1 or 2 carbon atoms; phenyl or phenoxy, in each case unsubstituted or substituted by fluorine, chlorine or bromine, and

Ar' represents phenyl, furyl or pyridyl, in each case unsubstituted or monosubstituted to trisubstituted by identical or different substituents, or represents phenylalkyl which is unsubstituted or monosubstituted to trisubstituted in the phenyl moiety by identical or different substituents and has 1 or 2 carbon atoms in the alkyl moiety, suitable phenyl substituents in each case being the above-mentioned phenyl substituents.

**4.** Amino acid amide derivatives of the formula (I) according to Claim 1, in which

$R^1$ represents hydrogen and

$R^2$ represents hydrogen or methyl and

$R^3$ and $R^4$ are identical or different and represent hydrogen, methyl, ethyl, n- or i-propyl, n-, i-, s- or t-butyl or 3-pentyl or

$R^3$ and $R^4$, together with the carbon atom to which they are bonded, form a cyclopropyl, cyclopentyl or cyclohexyl ring and

$R^5$ represents hydrogen or methyl and

$R^6$ represents hydrogen, methyl or ethyl and

Ar represents phenyl which is unsubstituted, or monosubstituted or disubstituted by identical or different substituents, suitable substituents being the following: methyl, ethyl, n- and i-propyl, n-, i-, s- and t-butyl, methoxy, ethoxy, i- and n-propoxy, n-, i-, s-and t-butoxy, trifluoromethyl, difluoromethyl, pentafluoroethyl, tetrafluoroethyl, trifluorochloroethyl, trifluoroethyl, trifluoroethoxy, difluoromethoxy, pentafluoroethoxy, tetrafluoroethoxy, trifluorochloroethoxy, trifluoromethoxy and trifluoromethylthio; chlorine, bromine, fluorine, nitro and cyano, and

Ar' represents benzyl, 1,2-phenethyl or phenyl which are unsubstituted, or monosubstituted or disubstituted in the phenyl moiety in each case by identical or different substituents, suitable phenyl substituents in each case being the abovementioned phenyl substituents.

**5.** Amino acid amide derivatives of the general formula (I) according to Claim 1, in which

$R^1$, $R^2$, $R^3$, $R^6$, Ar and Ar' have the meanings indicated in one of Claims 1 to 4 and

$R^3$ represents hydrogen and

$R^4$ represents methyl, ethyl, n-propyl, t-butyl, i-propyl, i-butyl, s-butyl or 3-pentyl, or

$R^3$ and $R^4$, together with the carbon atom to which they are bonded, form a cycloalkyl ring having 3 to 6 carbon atoms.

**6.** Process for the preparation of amino acid amide derivatives of the general formula (I)

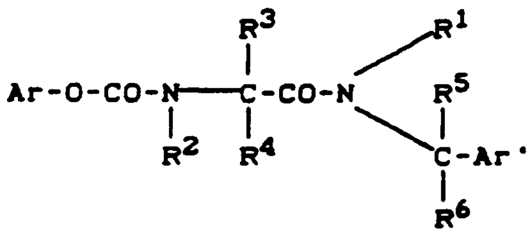

(I)

in which

| | |
|---|---|
| Ar and Ar' | are identical or different and in each case represent unsubstituted or substituted aryl, aralkyl, heteroaryl or heteroarylalkyl, |
| $R^1$, $R^2$, $R^3$, $R^4$, $R^5$ and $R^6$ | are identical or different and represent hydrogen or alkyl or |
| $R^3$ and $R^4$, | together with the carbon atom to which they are bonded, form a cycloalkyl ring, |

characterized in that

a substituted amino acid of the formula (II)

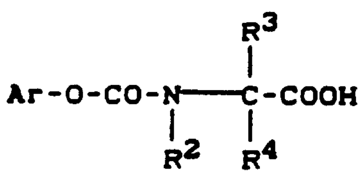

(II)

in which

Ar, $R^2$, $R^3$ and $R^4$ have the abovementioned meaning or their carboxyl-activated derivatives,

if appropriate in the presence of a catalyst, if appropriate in the presence of an acid-binding agent and if appropriate in the presence of a diluent

is reacted with an amine of the formula (III)

$HNR^1\text{-}CR^5R^6Ar'$ (III)

in which

Ar', $R^1$, $R^5$ and $R^6$ have the abovementioned meaning.

**7.** Pest-combating agents, characterized in that they contain at least one amino acid amide derivative of the formula (I) according to Claims 1 to 6.

**8.** Use of amino acid amide derivatives of the formula (I) according to Claims 1 to 6 for combating pests.

**9.** Method for combating pests, characterized in that amino acid amide derivatives of the formula (I) according to Claims 1 to 6 are allowed to act on pests and/or their environment.

**10.** Method for the production of pest-combating agents, characterized in that amino acid amide derivatives of the formula (I) according to Claims 1 to 6 are mixed with extenders and/or surface-active agents.

**Claims for the following Contracting State : ES**

**1.** Process for the preparation of amino acid amide derivatives of the general formula (I)

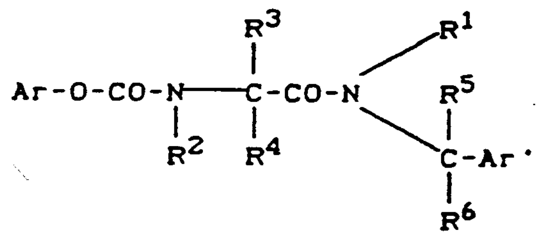

in which

Ar and Ar' — are identical or different and in each case represent unsubstituted or substituted aryl, aralkyl, heteroaryl or heteroarylalkyl,

$R^1$, $R^2$, $R^3$, $R^4$, $R^5$ and $R^6$ — are identical or different and represent hydrogen or alkyl or

$R^3$ and $R^4$, — together with the carbon atom to which they are bonded, form a cycloalkyl ring,

characterized in that
a substituted amino acid of the formula (II)

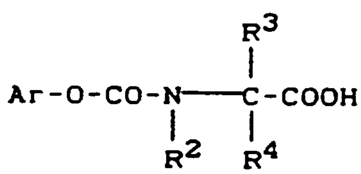

(II)

in which

Ar, $R^2$, $R^3$ and $R^4$ have the abovementioned meaning or their carboxyl-activated derivatives,
if appropriate in the presence of a catalyst, if appropriate in the presence of an acid-binding agent and if appropriate in the presence of a diluent
is reacted with an amine of the formula (III)

$HNR^1$-$CR^5R^6Ar'$ (III)

in which
Ar', $R^1$, $R^5$ and $R^6$ have the abovementioned meaning.

**2.** Process for the preparation of amino acid amide derivatives of the formula (I) according to Claim 1, in which

$R^1$, $R^2$, $R^3$, $R^4$, $R^5$ and $R^6$ — are identical or different and represent hydrogen or straight-chain or branched alkyl having 1 to 6 carbon atoms or

$R^3$ and $R^4$, — together with the carbon atom to which they are bonded, form a cycloalkyl ring having 3 to 7 carbon atoms and

Ar and Ar' — are identical or different and represent phenyl, furyl and pyridyl, in each case unsubstituted or substituted, or phenylalkyl which is unsubstituted or substituted in the phenyl moiety and which has 1 to 4 carbon atoms in the straight-chain or branched alkyl moiety, suitable substituents in the phenyl moiety in each case being:
alkyl, alkoxy and alkylthio each having 1 to 4 carbon atoms; halogenoalkyl, halogenoalkoxy and halogenoalkylthio each having 1 to 4 carbon atoms and 1 to 9 halogen atoms, the halogen atoms being identical or different; hydroxyl; halogen; cyano; nitro; dialkylamino

having 1 to 4 carbon atoms per alkyl group; carboxyl; alkylalkoxy having 1 to 4 carbon atoms in each alkyl moiety; carbonylalkoxy having 1 to 4 carbon atoms in the alkyl moiety; carbonylalkyl having 1 to 4 carbon atoms in the alkyl moiety; formyl; carbonylaryloxy having 5 to 10 carbon atoms in the aryl moiety; carbonylaryl having 6 to 10 carbon atoms in the aryl moiety; oxycarbonylalkyl having 1 to 4 carbon atoms in the alkyl moiety; oxycarbonylaryl having 6 to 10 carbon atoms in the aryl moiety; carbonylamino, carbonylaminoalkyl, carbonylaminodialkyl, aminocarbonyl, alkylaminocarbonyl, aminocarbonylalkyl and alkylaminocarbonylalkyl each having 1 to 4 carbon atoms in the alkyl moiety; sulphonamido; sulphonalkyl; sulphonylalkyl and sulphonylalkoxy each having 1 to 4 carbon atoms; phenyl or phenoxy, in each case unsubstituted or substituted by halogen.

**3.** Process for the preparation of amino acid amide derivatives of the formula (I) according to Claim 1, in which

| | |
|---|---|
| $R^1$ | represents hydrogen and |
| $R^2$, $R^3$ and $R^4$ | are identical or different and represent hydrogen or straight-chain or branched alkyl having 1 to 5 carbon atoms or |
| $R^3$ and $R^4$, | together with the carbon atom to which they are bonded, form a cycloalkyl ring having 3 to 6 carbon atoms and |
| $R^5$ and $R^6$ | are identical or different and represent hydrogen, methyl or ethyl and |
| Ar | represents phenyl which is unsubstituted or monosubstituted to trisubstituted by identical or different substituents, suitable substituents being the following: alkyl, alkoxy and alkylthio each having 1 or 2 carbon atoms; halogenoalkyl, halogenoalkoxy and halogenoalkylthio having 1 or 2 carbon atoms and 1 to 5 halogen atoms, the halogen atoms being identical or different; hydroxyl; fluorine, chlorine, bromine and iodine; cyano; nitro; dialkylamino having 1 or 2 carbon atoms per alkyl group; carboxyl; alkylalkoxy having 1 or 2 carbon atoms in each alkyl moiety; carbonylalkoxy having 1 or 2 carbon atoms in the alkyl moiety; carbonylalkyl having 1 or 2 carbon atoms in the alkyl moiety; formyl; carbonylphenoxy; benzoyl; oxycarbonylalkyl having 1 or 2 carbon atoms; benzoyloxy; carbonylamino, carbonylaminoalkyl, carbonylaminodialkyl, aminocarbonyl, alkylaminocarbonyl, aminocarbonylalkyl and alkylaminocarbonylalkyl each having 1 or 2 carbon atoms in the alkyl moiety; sulphonamido; sulphonalkyl, sulphonylalkyl and sulphonylalkoxy each having 1 or 2 carbon atoms; phenyl or phenoxy, in each case unsubstituted or substituted by fluorine, chlorine or bromine, and |
| Ar' | represents phenyl, furyl or pyridyl, in each case unsubstituted or monosubstituted to trisubstituted by identical or different substituents, or represents phenylalkyl which is unsubstituted or monosubstituted to trisubstituted in the phenyl moiety by identical or different substituents and has 1 or 2 carbon atoms in the alkyl moiety, suitable phenyl substituents in each case being the above-mentioned phenyl substituents. |

**4.** Process for the preparation of amino acid amide derivatives of the formula (I), according to Claim 1, in which

| | |
|---|---|
| $R^1$ | represents hydrogen and |
| $R^2$ | represents hydrogen or methyl and |
| $R^3$ and $R^4$ | are identical or different and represent hydrogen, methyl, ethyl, n- or i-propyl, n-, i-, s- or t-butyl or 3-pentyl or |
| $R^3$ and $R^4$, | together with the carbon atom to which they are bonded, form a cyclopropyl, cyclopentyl or cyclohexyl ring and |
| $R^5$ | represents hydrogen or methyl and |
| $R^6$ | represents hydrogen, methyl or ethyl and |
| Ar | represents phenyl which is unsubstituted, or monosubstituted or disubstituted by identical or different substituents, suitable substituents being the following: methyl, ethyl, n- and i-propyl, n-, i-, s- and t-butyl, methoxy, ethoxy, i- and n-propoxy, n-, i-, s-and t-butoxy, trifluoromethyl, difluoromethyl, pentafluoroethyl, tetrafluoroethyl, |

trifluorochloroethyl, trifluoroethyl, trifluoroethoxy, difluoromethoxy, pentafluoroethoxy, tetrafluoroethoxy, trifluorochloroethoxy, trifluoromethoxy and trifluoromethylthio; chlorine, bromine, fluorine, nitro and cyano, and

Ar' represents benzyl, 1,2-phenethyl or phenyl which are unsubstituted, or monosubstituted or disubstituted in the phenyl moiety in each case by identical or different substituents, suitable phenyl substituents in each case being the abovementioned phenyl substituents.

**5.** Process for the preparation of amino acid amide derivatives of the general formula (I) according to Claim 1, in which

$R^1$, $R^2$, $R^5$, $R^6$, Ar and Ar' have the meanings indicated in one of Claims 1 to 4 and

$R^3$ represents hydrogen and

$R^4$ represents methyl, ethyl, n-propyl, t-butyl, i-propyl, i-butyl, s-butyl or 3-pentyl, or

$R^3$ and $R^4$, together with the carbon atom to which they are bonded, form a cycloalkyl ring having 3 to 6 carbon atoms.

**6.** Pest-combating agents, characterized in that they contain at least one amino acid amide derivative of the formula (I) according to Claims 1 to 5.

**7.** Use of amino acid amide derivatives of the formula (I) according to Claims 1 to 5 for combating pests.

**8.** Method for combating pests, characterized in that amino acid amide derivatives of the formula (I) according to Claims 1 to 5 are allowed to act on pests and/or their environment.

**9.** Method for the production of pest-combating agents, characterized in that amino acid amide derivatives of the formula (I) according to Claims 1 to 5 are mixed with extenders and/or surface-active agents.

**Revendications**

**Revendications pour les Etats contractants suivants : BE, CH, DE, DK, FR, GB, GR, IT, LI, NL**

**1.** Dérivés d'amides d'aminoacides répondant à la formule générale (I)

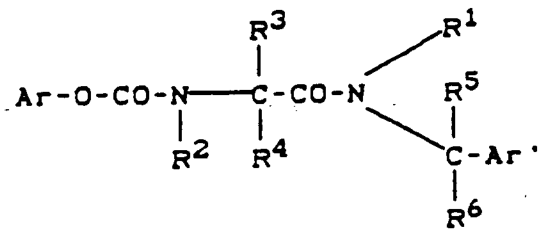 (I),

dans laquelle

Ar et Ar' sont identiques ou différents et représentent un groupe aryle non substitué ou substitué, un groupe aralkyle non substitué ou substitué, un groupe hétéroaryle non substitué ou substitué et un groupe hétéroarylalkyle non substitué ou substitué,

$R^1$, $R^2$, $R^3$, $R^4$, $R^5$ et $R^6$ sont identiques ou différents et représentent un atome d'hydrogène ou un groupe alkyle, ou bien

$R^3$ et $R^4$ forment, conjointement avec l'atome de carbone auquel ils sont liés, un noyau cycloalkyle.

**2.** Dérivés d'amides d'aminoacides de formule (I) selon la revendication 1, dans lesquels

$R^1$, $R^2$, $R^3$, $R^4$, $R^5$ et $R^6$ sont identiques ou différents et représentent un atome d'hydrogène; ou un groupe alkyle à chaîne droite ou ramifiée contenant de 1 à 6 atomes de carbone, ou bien

$R^3$ et $R^4$ forment, conjointement avec l'atome de carbone auquel ils sont liés, un

noyau cycloalkyle contenant de 3 à 7 atomes de carbone, et

Ar et Ar' sont identiques ou différents et représentent un groupe phényle, un groupe furyle et un groupe pyridyle respectivement non substitués ou substitués; ou bien un groupe phénylalkyle non substitué ou substitué dans la fraction phényle contenant de 1 à 4 atomes de carbone dans la fraction alkyle à chaîne droite ou ramifiée, dans lequel, comme substituants dans la fraction phényle, entrent respectivement en ligne de compte :

un groupe alkyle, un groupe alcoxy et un groupe alkylthio contenant chacun de 1 à 4 atomes de carbone; un groupe halogénalkyle, un groupe halogénalcoxy et un groupe halogénalkylthio contenant chacun de 1 à 4 atomes de carbone et de 1 à 9 atomes d'halogène, les atomes d'halogène étant identiques ou différents; un groupe hydroxyle; un atome d'halogène; un groupe cyano; un groupe nitro; un groupe dialkylamino contenant de 1 à 4 atomes de carbone dans chaque groupe alkyle; un groupe carboxyle; un groupe alkylalcoxy contenant de 1 à 4 atomes de carbone dans chaque fraction alkyle; un groupe carbonylalcoxy contenant de 1 à 4 atomes de carbone dans la fraction alkyle; un groupe carbonylalkyle contenant de 1 à 4 atomes de carbone dans la fraction alkyle; un groupe formyle; un groupe carbonylaryloxy contenant de 5 à 10 atomes de carbone dans la fraction aryle; un groupe carbonylaryle contenant de 6 à 10 atomes de carbone dans la fraction aryle; un groupe oxycarbonylalkyle contenant de 1 à 4 atomes de carbone dans la fraction alkyle; un groupe oxycarbonylaryle contenant de 6 à 10 atomes de carbone dans la fraction aryle; un groupe carbonylamino; un groupe carbonylaminoalkyle, un groupe carbonylaminodialkyle, un groupe aminocarbonyle, un groupe alkylaminocarbonyle, un groupe aminocarbonylalkyle et un groupe alkylaminocarbonylalkyle contenant respectivement de 1 à 4 atomes de carbone dans la fraction alkyle; un groupe sulfonamido; un groupe sulfonalkyle; un groupe sulfonylalkyle et un groupe sulfonylalcoxy contenant chacun de 1 à 4 atomes de carbone; un groupe phényle ou un groupe phénoxy respectivement non substitués ou substitués par un atome d'halogène.

**3.** Dérivés d'amides d'aminoacides de formule (I) selon la revendication 1, dans lesquels

$R^1$ représente un atome d'hydrogène, et

$R^2$, $R^3$ et $R^4$ sont identiques ou différents et représentent un atome d'hydrogène; ou un groupe alkyle à chaîne droite ou ramifiée contenant de 1 à 5 atomes de carbone, ou bien

$R^3$ et $R^4$ forment, conjointement avec l'atome de carbone auquel ils sont liés, un noyau cycloalkyle contenant de 3 à 6 atomes de carbone, et

$R^5$ et $R^6$ sont identiques ou différents et représentent un atome d'hydrogène, un groupe méthyle ou un groupe éthyle, et

Ar représente un groupe phényle non substitué ou substitué de 1 à 3 fois de manière identique ou différente, dans lequel entrent en ligne de compte, les substituants ci-après :

un groupe alkyle, un groupe alcoxy et un groupe alkylthio contenant chacun 1 ou 2 atomes de carbone; un groupe halogénalkyle, un groupe halogénalcoxy et un groupe halogénalkylthio contenant 1 ou 2 atomes de carbone et de 1 à 5 atomes d'halogène, les atomes d'halogène étant identiques ou différents; un groupe hydroxyle; un atome de fluor, un atome de chlore, un atome de brome et un atome d'iode; un groupe cyano; un groupe nitro; un groupe dialkylamino contenant 1 ou 2 atomes de carbone dans chaque groupe alkyle; un groupe carboxyle; un groupe alkylalcoxy contenant 1 ou 2 atomes de carbone dans chaque fraction alkyle; un groupe carbonylalcoxy contenant 1 ou 2 atomes de carbone dans la fraction alkyle; un groupe carbonylalkyle contenant 1 ou 2 atomes de carbone dans la fraction alkyle; un groupe formyle; un groupe carbonylphénoxy; un groupe benzoyle; un groupe oxycarbonylalkyle contenant 1 ou 2 atomes de carbone; un groupe benzoyloxy; un groupe carbonylamino, un groupe carbonylaminoalkyle, un groupe carbonylaminodialkyle, un groupe aminocarbonyle, un groupe alkylaminocarbonyle, un groupe aminocarbonylalkyle et un groupe alkylaminocarbonylalkyle contenant respectivement 1 ou 2 atomes de carbone dans la fraction alkyle; un groupe sulfonamido; un groupe sulfonalkyle, un groupe sulfonylalkyle et un groupe sulfonylalcoxy contenant respectivement 1 ou 2 atomes de carbone; un groupe phényle ou un groupe phénoxy respectivement non substitués ou substitués par un atome de fluor, par un atome de chlore ou par un atome de brome; et

Ar' représente un groupe phényle, un groupe furyle ou un groupe pyridyle respectivement non substitués ou substitués de 1 à 3 fois de manière identique ou différente; ou encore un groupe phénylalkyle non substitué ou substitué de 1 à 3 fois de manière identique ou différente dans la fraction alkyle et contenant 1 ou 2 atomes de carbone dans la fraction alkyle, dans lequel, comme substituants du groupe phényle, entrent en ligne de compte respectivement les substituants du groupe phényle mentionnés ci-dessus.

**4.** Dérivés d'amides d'aminoacides de formule (I) selon la revendication 1, dans lesquels

$R^1$ représente un atome d'hydrogène, et

$R^2$ représente un atome d'hydrogène ou un groupe méthyle, et

$R^3$ et $R^4$ sont identiques ou différents et représentent un atome d'hydrogène, un groupe méthyle, un groupe éthyle, un groupe n- ou i-propyle, un groupe n-, i-, s- ou t-butyle ou un groupe 3-pentyle, ou bien $R^3$ et $R^4$ forment, conjointement avec l'atome de carbone auquel ils sont liés, un noyau cyclopropyle, un noyau cyclopentyle ou un noyau cyclohexyle, et

$R^5$ représente un atome d'hydrogène ou un groupe méthyle, et

$R^6$ représente un atome d'hydrogène, un groupe méthyle ou un groupe éthyle, et

Ar représente un groupe phényle non substitué ou substitué de 1 à 2 fois de manière identique ou différente, dans lequel entrent en ligne de compte, les substituants ci-après : un groupe méthyle, un groupe éthyle, un groupe n- et i-propyle, un groupe n-, i-, s- et t-butyle, un groupe méthoxy, un groupe éthoxy, un groupe i- et n-propoxy, un groupe n-, i-, s- et t-butoxy, un groupe trifluorométhyle, un groupe difluorométhyle, un groupe pentafluoréthyle, un groupe tétrafluoréthyle, un groupe trifluorochloréthyle, un groupe trifluoréthyle, un groupe trifluoréthoxy, un groupe difluorométhoxy, un groupe pentafluoréthoxy, un groupe tétrafluoréthoxy, un groupe trifluorochloréthoxy, un groupe trifluorométhoxy et un groupe trifluorométhylthio; un atome de chlore, un atome de brome, un atome de fluor, un groupe nitro et un groupe cyano;

Ar' représente un groupe benzyle, un groupe 1,2-phénéthyle ou un groupe phényle respectivement non substitués ou substitués dans la fraction phényle de 1 à 2 fois de manière identique ou différente, dans lesquels, comme substituants du groupe phényle, entrent respectivement en ligne de compte les substituants du groupe phényle susmentionnés.

**5.** Dérivés d'amides d'aminoacides de formule (I) selon la revendication 1, dans lesquels

$R^1$, $R^2$, $R^5$, $R^6$, Ar et Ar' ont les significations indiquées dans une des revendications 1 à 4, et

$R^3$ représente un atome d'hydrogène, et

$R^4$ représente un groupe méthyle, un groupe éthyle, un groupe n-propyle, un groupe t-butyle, un groupe i-propyle, un groupe i-butyle, un groupe s-butyle ou un groupe 3-pentyle, ou bien

$R^3$ et $R^4$ forment, conjointement avec l'atome de carbone auquel ils sont liés, un noyau cycloalkyle contenant de 3 à 6 atomes de carbone.

**6.** Procédé pour la préparation de dérivés d'amides d'aminoacides répondant à la formule générale (I)

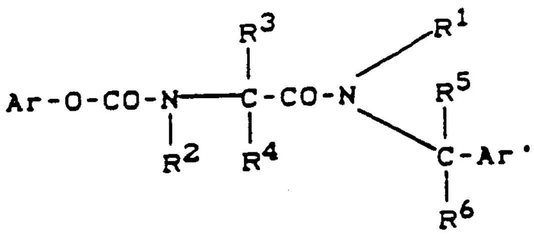

(I),

dans laquelle

| | |
|---|---|
| Ar et Ar' | sont identiques ou différents et représentent un groupe aryle, un groupe aralkyle, un groupe hétéroaryle ou un groupe hétéroarylalkyle respectivement non substitués ou substitués, |
| $R^1$, $R^2$, $R^3$, $R^4$, $R^5$ et $R^6$ | sont identiques ou différents et représentent un atome d'hydrogène ou un groupe alkyle, ou bien |
| $R^3$ et $R^4$ | forment, conjointement avec l'atome de carbone auquel ils sont liés, un noyau cycloalkyle, |

caractérisé en ce qu'on fait réagir un aminoacide substitué de formule (II)

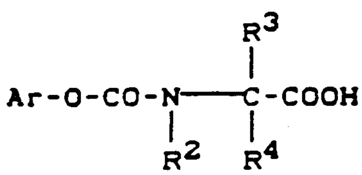

(II),

dans laquelle

Ar, $R^2$, $R^3$ et $R^4$ ont la signification indiquée ci-dessus, ou bien leurs dérivés carboxy-activés, éventuellement en présence d'un catalyseur, éventuellement en présence d'un agent liant les acides et éventuellement en présence d'un diluant,

avec une amine de formule (III)

$HNR^1$-$CR^5R^6Ar'$ (III)

dans laquelle

Ar', $R^1$, $R^5$ et $R^6$ ont la signification indiquée ci-dessus.

**7.** Agents de lutte contre les parasites, caractérisés par une teneur en au moins un dérivé d'amide d'aminoacide de formule (I) selon les revendications 1 à 6.

**8.** Utilisation de dérivés d'amides d'aminoacides de formule (I) selon les revendications 1 à 6, pour lutter contre les parasites.

**9.** Procédé pour lutter contre les parasites, caractérisé en ce qu'on laisse agir des dérivés d'amides d'aminoacides de formule (I) selon les revendications 1 à 6, sur des parasites et/ou sur leur biotope.

**10.** Procédé pour la préparation d'agents de lutte contre les parasites, caractérisé en ce qu'on mélange des dérivés d'amides d'aminoacides de formule (I) selon les revendications 1 à 6, avec des diluants et/ou des agents tensioactifs.

**Revendications pour l'Etat contractant suivant : ES**

1. Procédé pour la préparation de dérivés d'amides d'aminoacides répondant à la formule générale (I)

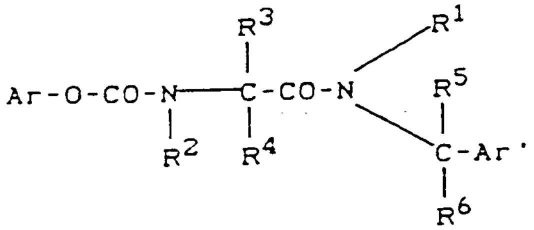

(I),

dans laquelle

| | |
|---|---|
| Ar et Ar' | sont identiques ou différents et représentent un groupe aryle, un groupe aralkyle, un groupe hétéroaryle ou un groupe hétéroarylalkyle respectivement non substitués ou substitués, |
| $R^1$, $R^2$, $R^3$, $R^4$, $R^5$ et $R^6$ | sont identiques ou différents et représentent un atome d'hydrogène ou un groupe alkyle, ou bien |
| $R^3$ et $R^4$ | forment, conjointement avec l'atome de carbone auquel ils sont liés, un noyau cycloalkyle, |

caractérisé en ce qu'on fait réagir un aminoacide substitué de formule (II)

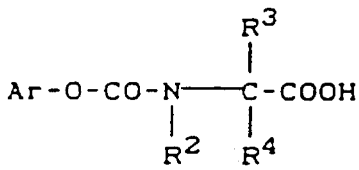

(II),

dans laquelle

Ar, $R^2$, $R^3$ et $R^4$ ont la signification indiquée ci-dessus, ou bien leurs dérivés carboxy-activés, éventuellement en présence d'un catalyseur, éventuellement en présence d'un agent liant les acides et éventuellement en présence d'un diluant,

avec une amine de formule (III)

$HNR^1\text{-}CR^5R^6Ar'$ (III)

dans laquelle

Ar', $R^1$, $R^5$ et $R^6$ ont la signification indiquée ci-dessus.

2. Procédé selon la revendication 1, dans lequel

| | |
|---|---|
| $R^1$, $R^2$, $R^3$, $R^4$, $R^5$ et $R^6$ | sont identiques ou différents et représentent un atome d'hydrogène; ou un groupe alkyle à chaîne droite ou ramifiée contenant de 1 à 6 atomes de carbone, ou bien |
| $R^3$ et $R^4$ | forment, conjointement avec l'atome de carbone auquel ils sont liés, un noyau cycloalkyle contenant de 3 à 7 atomes de carbone, et |
| Ar et Ar' | sont identiques ou différents et représentent un groupe phényle, un groupe furyle et un groupe pyridyle respectivement non substitués ou substitués; ou bien un groupe phénylalkyle non substitué ou substitué dans la fraction phényle contenant de 1 à 4 atomes de carbone dans la fraction alkyle à chaîne droite ou ramifiée, dans lequel, comme substituants dans la fraction phényle; entrent respectivement en ligne de compte : un groupe alkyle, un groupe alcoxy et un groupe alkylthio contenant chacun de 1 à 4 atomes de carbone; un groupe halogénalkyle, un |

groupe halogénalcoxy et un groupe halogénalkylthio contenant chacun de 1 à 4 atomes de carbone et de 1 à 9 atomes d'halogène, les atomes d'halogène étant identiques ou différents; un groupe hydroxyle; un atome d'halogène; un groupe cyano; un groupe nitro; un groupe dialkylamino contenant de 1 à 4 atomes de carbone dans chaque groupe alkyle; un groupe carboxyle; un groupe alkylalcoxy contenant de 1 à 4 atomes de carbone dans chaque fraction alkyle; un groupe carbonylalcoxy contenant de 1 à 4 atomes de carbone dans la fraction alkyle; un groupe carbonylalkyle contenant de 1 à 4 atomes de carbone dans la fraction alkyle; un groupe formyle; un groupe carbonylaryloxy contenant de 5 à 10 atomes de carbone dans la fraction aryle; un groupe carbonylaryle contenant de 6 à 10 atomes de carbone dans la fraction aryle; un groupe oxycarbonylalkyle contenant de 1 à 4 atomes de carbone dans la fraction alkyle; un groupe oxycarbonylaryle contenant de 6 à 10 atomes de carbone dans la fraction aryle; un groupe carbonylamino; un groupe carbonylaminoalkyle, un groupe carbonylaminodialkyle, un groupe aminocarbonyle, un groupe alkylaminocarbonyle, un groupe aminocarbonylalkyle et un groupe alkylaminocarbonylalkyle contenant respectivement de 1 à 4 atomes de carbone dans la fraction alkyle; un groupe sulfonamido; un groupe sulfonalkyle; un groupe sulfonylalkyle et un groupe sulfonylalcoxy contenant chacun de 1 à 4 atomes de carbone; un groupe phényle ou un groupe phénoxy respectivement non substitués ou substitués par un atome d'halogène.

**3.** Procédé selon la revendication 1, dans lequel

| | |
|---|---|
| $R^1$ | représente un atome d'hydrogène, et |
| $R^2$, $R^3$ et $R^4$ | sont identiques ou différents et représentent un atome d'hydrogène; ou un groupe alkyle à chaîne droite ou ramifiée contenant de 1 à 5 atomes de carbone, ou bien |
| $R^3$ et $R^4$ | forment, conjointement avec l'atome de carbone auquel ils sont liés, un noyau cycloalkyle contenant de 3 à 6 atomes de carbone, et |
| $R^5$ et $R^6$ | sont identiques ou différents et représentent un atome d'hydrogène, un groupe méthyle ou un groupe éthyle, et |
| Ar | représente un groupe phényle non substitué ou substitué de 1 à 3 fois de manière identique ou différente, dans lequel entrent en ligne de compte, les substituants ci-après : un groupe alkyle, un groupe alcoxy et un groupe alkylthio contenant chacun 1 ou 2 atomes de carbone; un groupe halogénalkyle, un groupe halogénalcoxy et un groupe halogénalkylthio contenant 1 ou 2 atomes de carbone et de 1 à 5 atomes d'halogène, les atomes d'halogène étant identiques ou différents; un groupe hydroxyle; un atome de fluor, un atome de chlore, un atome de brome et un atome d'iode; un groupe cyano; un groupe nitro; un groupe dialkylamino contenant 1 ou 2 atomes de carbone dans chaque groupe alkyle; un groupe carboxyle; un groupe alkylalcoxy contenant 1 ou 2 atomes de carbone dans chaque fraction alkyle; un groupe carbonylalcoxy contenant 1 ou 2 atomes de carbone dans la fraction alkyle; un groupe carbonylalkyle contenant 1 ou 2 atomes de carbone dans la fraction alkyle; un groupe formyle; un groupe carbonylphénoxy; un groupe benzoyle; un groupe oxycarbonylalkyle contenant 1 ou 2 atomes de carbone; un groupe benzoyloxy; un groupe carbonylamino, un groupe carbonylaminoalkyle, un groupe carbonylaminodialkyle, un groupe aminocarbonyle, un groupe alkylaminocarbonyle, un groupe aminocarbonylalkyle et un groupe alkylaminocarbonylalkyle contenant respectivement 1 ou 2 atomes de carbone dans la fraction alkyle; un groupe sulfonamido; un groupe sulfonalkyle, un groupe sulfonylalkyle et un groupe sulfonylalcoxy contenant respectivement 1 ou 2 atomes de carbone; un groupe phényle ou un groupe phénoxy respectivement non substitués ou substitués par un atome de fluor, par un atome de chlore ou par un atome de brome; et |
| Ar' | représente un groupe phényle, un groupe furyle ou un groupe pyridyle respectivement non substitués ou substitués de 1 à 3 fois de manière identique ou différente; ou encore un groupe phénylalkyle non substitué ou substitué de 1 à 3 fois de |

manière identique ou différente dans la fraction alkyle et contenant 1 ou 2 atomes de carbone dans la fraction alkyle, dans lequel, comme substituants du groupe phényle, entrent en ligne de compte respectivement les substituants du groupe phényle mentionnés ci-dessus.

**4.** Procédé selon la revendication 1, dans lequel

| | |
|---|---|
| $R^1$ | représente un atome d'hydrogène, et |
| $R^2$ | représente un atome d'hydrogène ou un groupe méthyle, et |
| $R^3$ et $R^4$ | sont identiques ou différents et représentent un atome d'hydrogène, un groupe méthyle, un groupe éthyle, un groupe n- ou i-propyle, un groupe n-, i-, s- ou t-butyle ou un groupe 3-pentyle, ou bien $R^3$ et $R^4$ forment, conjointement avec l'atome de carbone auquel ils sont liés, un noyau cyclopropyle, un noyau cyclopentyle ou un noyau cyclohexyle, et |
| $R^5$ | représente un atome d'hydrogène ou un groupe méthyle, et |
| $R^6$ | représente un atome d'hydrogène, un groupe méthyle ou un groupe éthyle, et |
| Ar | représente un groupe phényle non substitué ou substitué de 1 à 2 fois de manière identique ou différente, dans lequel entrent en ligne de compte, les substituants ci-après : un groupe méthyle, un groupe éthyle, un groupe n- et i-propyle, un groupe n-, i-, s- et t-butyle, un groupe méthoxy, un groupe éthoxy, un groupe i- et n-propoxy, un groupe n-, i-, s- et t-butoxy, un groupe trifluorométhyle, un groupe difluorométhyle, un groupe pentafluoréthyle, un groupe tétrafluoréthyle, un groupe trifluorochloréthyle, un groupe trifluoréthyle, un groupe trifluoréthoxy, un groupe difluorométhoxy, un groupe pentafluoréthoxy, un groupe tétrafluoréthoxy, un groupe trifluorochloréthoxy, un groupe trifluorométhoxy et un groupe trifluorométhylthio; un atome de chlore, un atome de brome, un atome de fluor, un groupe nitro et un groupe cyano; |
| Ar' | représente un groupe benzyle, un groupe 1,2-phénéthyle ou un groupe phényle respectivement non substitués ou substitués dans la fraction phényle de 1 à 2 fois de manière identique ou différente, dans lesquels, comme substituants du groupe phényle, entrent respectivement en ligne de compte les substituants du groupe phényle susmentionnés. |

**5.** Procédé selon la revendication 1, dans lequel

| | |
|---|---|
| $R^1$, $R^2$, $R^5$, $R^6$, Ar et Ar' | ont les significations indiquées dans une des revendications 1 à 4, et |
| $R^3$ | représente un atome d'hydrogène, et |
| $R^4$ | représente un groupe méthyle, un groupe éthyle, un groupe n-propyle, un groupe t-butyle, un groupe i-propyle, un groupe i-butyle, un groupe s-butyle ou un groupe 3-pentyle, ou bien |
| $R^3$ et $R^4$ | forment, conjointement avec l'atome de carbone auquel ils sont liés, un noyau cycloalkyle contenant de 3 à 6 atomes de carbone. |

**6.** Agents de lutte contre les parasites, caractérisés par une teneur en au moins un dérivé d'amide d'aminoacide de formule (I) selon les revendications 1 à 5.

**7.** Utilisation de dérivés d'amides d'aminoacides de formule (I) selon les revendications 1 à 5, pour lutter contre les parasites.

**8.** Procédé pour lutter contre les parasites, caractérisé en ce qu'on laisse agir des dérivés d'amides d'aminoacides de formule (I) selon les revendications 1 à 5, sur des parasites et/ou sur leur biotope.

**9.** Procédé pour la préparation d'agents de lutte contre les parasites, caractérisé en ce qu'on mélange des dérivés d'amides d'aminoacides de formule (I) selon les revendications 1 à 5, avec des diluants et/ou des agents tensioactifs.